# EUROPEAN PATENT APPLICATION

(11) **EP 2 590 134 A1**
(43) Date of publication of application: **08.05.2013**
(21) Application number: 11800897.8
(22) Date of filing: 29.06.2011
(51) Int. Cl.: G06Q 50/00, A61B 5/00

(54) **INFECTION SPREAD PREVENTION SUPPORT SYSTEM, INFECTION SPREAD PREVENTION SUPPORT SERVER, EXAMINATION TERMINAL, MOBILE TERMINAL AND PROGRAM**

(30) Priority: 30.06.2010 JP 2010150330
(71) Applicant: Nikon Corporation, Tokyo 100-8331 (JP)
(72) Inventor: SHIONO Hirofumi, Tokyo 100-8331 (JP); NEI Masahiro, Tokyo 100-8331 (JP); INOUE Hideya, Tokyo 100-8331 (JP); YAMASHITA Ryoichi, Tokyo 100-8331 (JP); SHIBAYAMA Miyuki, Tokyo 100-8331 (JP)
(74) Representative: Viering, Jentschura & Partner
(86) International application number: PCT/JP2011/064914
(87) International publication number: WO 2012/002435

(57) **Abstract**

An infection spread prevention support system includes an examination terminal and an infection spread prevention support server. The examination terminal includes an examination information acquisition unit that acquires examination information to examine the infection of an infectious disease in subjects and a transmission and reception unit that transmits the examination information to the infection spread prevention support server. The infection spread prevention support server includes a receiving unit that receives examination information, an infection determination unit that determines the presence or degree of infection of the infectious disease using the examination information, an activity regulation information generation unit that generates activity regulation information to regulate the an activity of the infected patient based on infection determination information, which is a determination result of the infection determination unit, and information regarding the subjects, and an output unit that outputs the activity regulation information.

## Description

### Technical Field

The present invention relates to an infection spread prevention support system, an infection spread prevention support server, an examination terminal, a mobile terminal, and a program.
Priority is claimed on Japanese Patent Application No. 2010-150330, filed June 30, 2010, the content of which is incorporated herein by reference.

### Background Art

Conventionally, a remote examination system and a remote examination method that are executed outside a specialized institute, such as a hospital, have been known (for example, refer to Patent Document 1).

### Citation List

### Patent Literature

[Patent Document 1] Japanese Unexamined Patent Application, First Publication No. 2009-258886

### Summary of Invention

### Technical Problem

In the related art, however, there is a problem in that the spread of infectious diseases cannot be prevented even if a subject is infected with an infectious disease can be examined outside a specialized institute.
According to aspects related to the present invention, techniques capable of supporting the prevention of the spread of infectious diseases are provided.

### Solution to Problem

According to an aspect of the present invention, an infection spread prevention support system includes an examination terminal and a server. The examination terminal includes: an examination information acquisition unit that acquires examination information to examine infection of an infectious disease in subjects; and a transmission unit that transmits the examination information to the server. The server includes: a receiving unit that receives the examination information and information regarding the subjects; an infection determination unit configured to determine presence or degree of infection of the infectious disease by comparing the examination information with examination determination information stored in a storage unit; an activity regulation information generation unit configured to specify an infected patient of the infectious disease among the subjects based on infection determination information, which is a determination result of the infection determination unit, and the information regarding the subjects and to generate activity regulation information to regulate an activity of the infected patient; and an output unit that outputs the activity regulation information of the infected patient.

According to another aspect of the present invention, an infection spread prevention support system includes an examination terminal and a server. The examination terminal includes: an examination information acquisition unit that acquires examination information to examine infection of an infectious disease in subjects; an infection determination unit configured to determine presence or degree of infection of the infectious disease by comparing the examination information with examination determination information stored in a storage unit; and a transmission unit that transmits infection determination information, which is a determination result of the infection determination unit, to the server. The server includes: a receiving unit that receives the infection determination information and information regarding the subjects; an activity regulation information generation unit configured to specify an infected patient of the infectious disease among the subjects based on the infection determination information and the information regarding the subjects and to generate activity regulation information to regulate an activity of the infected patient; and an output unit that outputs the activity regulation information of the infected patient.

According to still another aspect of the present invention, an infection spread prevention support system includes an examination terminal and a server. The examination terminal includes: an examination information acquisition unit that acquires examination information to examine infection of an infectious disease in subjects; and a transmission unit that transmits the examination information to the server. The server includes: a receiving unit that receives the examination information and information regarding the subjects; an infection determination unit configured to determine the presence or degree of infection of the infectious disease by comparing the examination information with examination determination information stored in a storage unit; a treatment support information generation unit configured to specify an infected patient of the infectious disease among the subjects based on infection determination information, which is a determination result of the infection determination unit, and the information regarding the subjects and to generate treatment support information to support treatment of the infected patient; and an output unit that outputs the treatment support information of the infected patient.

According to still another aspect of the present invention, an infection spread prevention support system includes an examination terminal and a server. The examination terminal includes: an examination information acquisition unit that acquires examination information to examine infection of an infectious disease in subjects; an infection determination unit configured to determine presence or degree of infection of the infectious disease by comparing the examination information with examination determination information stored in a storage unit; and a transmission unit that transmits infection determination information, which is a determination result of the infection determination unit, to the server. The server includes: a receiving unit that receives the infection determination information and information regarding the subjects; a treatment support information generation unit configured to specify an infected patient of the infectious disease among the subjects based on the infection determination information and the information regarding the subjects and to generate treatment support information to support treatment of the infected patient; and an output unit that outputs the treatment support information of the infected patient.

According to still another aspect of the present invention, an infection spread prevention support system includes an examination terminal and a server. The examination terminal includes: an examination information acquisition unit that acquires examination information to examine infection of an infectious disease in subjects; and a transmission unit that transmits the examination information to the server. The server includes: a receiving unit that receives the examination information and information regarding the subjects; an infection determination unit configured to determine presence or degree of infection of the infectious disease by comparing the examination information with examination determination information stored in a storage unit; a life-sustaining support information generation unit configured to specify an infected patient of the infectious disease among the subjects based on infection determination information, which is a determination result of the infection determination unit, and the information regarding the subjects and to generate life-sustaining support information to support maintenance of life of the infected patient; and an output unit that outputs the life-sustaining support information of the infected patient.

According to still another aspect of the present invention, an infection spread prevention support system includes an examination terminal and a server. The examination terminal includes: an examination information acquisition unit that acquires examination information to examine infection of an infectious disease in subjects; an infection determination unit configured to determine presence or degree of infection of the infectious disease by comparing the examination information with examination determination information stored in a storage unit; and a transmission unit that transmits infection determination information, which is a determination result of the infection determination unit, to the server. The server includes: a receiving unit that receives the infection determination information and information regarding the subjects; a life-sustaining support information generation unit configured to specify an infected patient of the infectious disease among the subjects based on the infection determination information and the information regarding the subjects and to generate life-sustaining support information to support maintenance of life of the infected patient; and an output unit that outputs the life-sustaining support information of the infected patient.

According to still another aspect of the present invention, an infection spread prevention support server includes: a receiving unit that receives examination information to examine infection of an infectious disease in subjects and information regarding the subjects; an infection determination unit configured to determine presence or degree of infection of the infectious disease by comparing the examination information with examination determination information stored in a storage unit; an activity regulation information generation unit configured to specify an infected patient of the infectious disease among the subjects based on infection determination information, which is a determination result of the infection determination unit, and the information regarding the subjects and to generate activity regulation information to regulate an activity of the infected patient; and an output unit that outputs the activity regulation information of the infected patient.

According to still another aspect of the present invention, an infection spread prevention support server includes: a receiving unit that receives infection determination information, which is a determination result when presence or degree of infection of an infectious disease is determined by comparing examination information to examine infection of the infectious disease in subjects with examination determination information stored in a storage unit, and information regarding the subjects; an activity regulation information generation unit configured to specify an infected patient of the infectious disease among the subjects based on the infection determination information and the information regarding the subjects and to generate activity regulation information to regulate an activity of the infected patient; and an output unit that outputs the activity regulation information of the infected patient.

According to still another aspect of the present invention, an infection spread prevention support server includes: a receiving unit that receives examination information to examine infection of an infectious disease in subjects and information regarding the subjects; an infection determination unit configured to determine presence or degree of infection of the infectious disease by comparing the examination information with examination determination information stored in a storage unit; a treatment support information generation unit configured to specify an infected patient of the infectious disease among the subjects based on infection determination information, which is a determination result of the infection determination unit, and the information regarding the subjects and to generate treatment support information to support treatment of the infected patient; and an output unit that outputs the treatment support information of the infected patient.

According to still another aspect of the present invention, an infection spread prevention support server includes: a receiving unit that receives infection determination information, which is a determination result when presence or degree of infection of an infectious disease is determined by comparing examination information to examine infection of the infectious disease in subjects with examination determination information stored in a storage unit, and information regarding the subjects; a treatment support information generation unit configured to specify an infected patient of the infectious disease among the subjects based on the infection determination information and the information regarding the subjects and to generate treatment support information to support treatment of the infected patient; and an output unit that outputs the treatment support information of the infected patient.

According to still another aspect of the present invention, an infection spread prevention support server includes: a receiving unit that receives examination information to examine infection of an infectious disease in subjects and information regarding the subjects; an infection determination unit configured to determine presence or degree of infection of the infectious disease by comparing the examination information with examination determination information stored in a storage unit; a life-sustaining support information generation unit configured to specify an infected patient of the infectious disease among the subjects based on infection determination information, which is a determination result of the infection determination unit, and the information regarding the subjects and to generate life-sustaining support information to support maintenance of life of the infected patient; and an output unit that outputs the life-sustaining support information of the infected patient.

According to still another aspect of the present invention, an infection spread prevention support server includes: a receiving unit that receives infection determination information, which is a determination result when presence or degree of infection of an infectious disease is determined by comparing examination information to examine infection of the infectious disease in subjects with examination determination information stored in a storage unit, and information regarding the subjects; a life-sustaining support information generation unit configured to specify an infected patient of the infectious disease among the subjects based on the infection determination information and the information regarding the subjects and to generate life-sustaining support information to support maintenance of life of the infected patient; and an output unit that outputs the life-sustaining support information of the infected patient.

According to still another aspect of the present invention, an examination terminal includes: an examination information acquisition unit that acquires examination information used to determine presence or degree of infection of an infectious disease of a subject; an infection determination unit configured to determine the presence or degree of infection of the infectious disease by comparing the examination information with examination determination information stored in a storage unit; and a transmission unit that transmits infection determination information, which is a determination result of the infection determination unit, to a server.

According to still another aspect of the present invention, a mobile terminal includes: a receiving unit that receives, from a server configured to generate activity regulation information to regulate an activity of an infected patient of an infectious disease among subjects, entry and stay regulation information to regulate entry or stay of the infected patient or entry and exit regulation support information to regulate entry and exit to and from a medical institution for the subjects, as the activity regulation information, based on infection determination information indicating presence or degree of infection of the infectious disease in the subjects; a storage unit that stores the activity regulation information; a permission determination unit configured to determine whether or not entry is permitted, whether or not stay is permitted, or whether or not entry and exit are permitted based on the activity regulation information; and a determination result notification unit that sends a notification of a determination result of the permission determination unit.

According to still another aspect of the present invention, a mobile terminal includes: a receiving unit that receives a determination result regarding whether or not entry is permitted, whether or not stay is permitted, or whether or not entry and exit are permitted, which is based on activity regulation information for regulating an activity of an infected patient that is generated by a server based on infection determination information indicating presence or degree of infection of an infectious disease in a subject; and a determination result notification unit that sends a notification of the determination result.

According to still another aspect of the present invention, there is provided a program causing a computer of an infection spread prevention support server to execute: a receiving step of receiving examination information to examine infection of an infectious disease in subjects and information regarding the subjects; an infection determination step of determining presence or degree of infection of the infectious disease by comparing the examination information with examination determination information stored in a storage unit; a step of specifying an infected patient of the infectious disease among the subjects based on infection determination information, which is a determination result in the infection determination step, and the information regarding the subjects; an activity regulation information generation step of generating activity regulation information to regulate an activity of the infected patient; and an output step of outputting the activity regulation information.

According to still another aspect of the present invention, there is provided a program causing a computer of an examination terminal having a mounting unit of an examination device that examines infection of an infectious disease in a subject to execute: an examination information acquisition step of acquiring examination information used to determine presence or degree of infection of the infectious disease; an infection determination step of determining the presence or degree of infection of the infectious disease by comparing the examination information with examination determination information stored in a storage unit; a transmission step of transmitting infection determination information, which is a determination result in the infection determination step, to a server; a receiving step of receiving from the server at least one of activity regulation information to regulate an activity of an infected patient of the infectious disease, treatment support information to support treatment of the infected patient, and life-sustaining support information to support maintenance of life of the infected patient; and an output step of outputting the information received in the receiving step.

According to still another aspect of the present invention, there is provided a program causing a computer of a mobile terminal to execute: an activity regulation information receiving step of receiving, from a server configured to generate activity regulation information to regulate an activity of an infected patient of an infectious disease, entry and stay regulation information to regulate entry or stay of the infected patient or entry and exit regulation support information to regulate entry and exit to and from a medical institution for a subject, as the activity regulation information, based on infection determination information indicating presence or degree of infection of the infectious disease in the subject; a storage step of storing the activity regulation information; a permission determination step of determining whether or not entry is permitted, whether or not stay is permitted, or whether or not entry and exit are permitted based on the activity regulation information; and a determination result notification step of sending a notification of a determination result in the permission determination step.

### Advantageous Effects of Invention

According to the aspects related to the present invention, it is possible to support the prevention of the spread of infectious diseases.

### Brief Description of Drawings

FIG. 1 is a conceptual diagram of the entire system including an infection spread prevention support system according to a first embodiment related to the present invention.
FIG. 2A is an explanatory diagram illustrating the infection spread prevention support system according to the first embodiment.
FIG. 2B is an explanatory diagram illustrating the infection spread prevention support system according to the first embodiment.
FIG. 2C is an explanatory diagram illustrating the infection spread prevention support system according to the first embodiment.
FIG. 2D is an explanatory diagram illustrating the infection spread prevention support system according to the first embodiment.
FIG. 2E is an explanatory diagram illustrating the infection spread prevention support system according to the first embodiment.
FIG. 3A is an explanatory diagram illustrating the infection spread prevention support system according to the first embodiment.
FIG. 3B is an explanatory diagram illustrating the infection spread prevention support system according to the first embodiment.
FIG. 4A is an explanatory diagram illustrating the infection spread prevention support system according to the first embodiment.
FIG. 4B is an explanatory diagram illustrating the infection spread prevention support system according to the first embodiment.
FIG. 4C is an explanatory diagram illustrating the infection spread prevention support system according to the first embodiment.
FIG. 4D is an explanatory diagram illustrating the infection spread prevention support system according to the first embodiment.
FIG. 4E is an explanatory diagram illustrating the infection spread prevention support system according to the first embodiment.
FIG. 5A is an explanatory diagram illustrating the infection spread prevention support system according to the first embodiment.
FIG. 5B is an explanatory diagram illustrating the infection spread prevention support system according to the first embodiment.
FIG. 6 is an explanatory diagram illustrating the infection spread prevention support system according to the first embodiment.
FIG. 7A is an explanatory diagram illustrating the infection spread prevention support system according to the first embodiment.
FIG. 7B is an explanatory diagram illustrating the infection spread prevention support system according to the first embodiment.
FIG. 7C is an explanatory diagram illustrating the infection spread prevention support system according to the first embodiment.
FIG. 7D is an explanatory diagram illustrating the infection spread prevention support system according to the first embodiment.
FIG. 8 is an explanatory diagram illustrating the infection spread prevention support system according to the first embodiment.
FIG. 9 is a flow chart showing an example of the flow of the process of the infection spread prevention support system in the present embodiment.
FIG. 10 is a conceptual diagram of the entire system including an infection spread prevention support system according to a second embodiment related to the present invention.
FIG. 11A is an explanatory diagram illustrating the infection spread prevention support system according to the second embodiment.
FIG. 11B is an explanatory diagram illustrating the infection spread prevention support system according to the second embodiment.
FIG. 11C is an explanatory diagram illustrating the infection spread prevention support system according to the second embodiment.
FIG. 11D is an explanatory diagram illustrating the infection spread prevention support system according to the second embodiment.
FIG. 11E is an explanatory diagram illustrating the infection spread prevention support system according to the second embodiment.
FIG. 11F is an explanatory diagram illustrating the infection spread prevention support system according to the second embodiment.
FIG. 11G is an explanatory diagram illustrating the infection spread prevention support system according to the second embodiment.
FIG. 11H is an explanatory diagram illustrating the infection spread prevention support system according to the second embodiment.
FIG. 12A is an explanatory diagram illustrating the infection spread prevention support system according to the second embodiment.
FIG. 12B is an explanatory diagram illustrating the infection spread prevention support system according to the second embodiment.
FIG. 12C is an explanatory diagram illustrating the infection spread prevention support system according to the second embodiment.
FIG. 13 is a conceptual diagram of the entire system including an infection spread prevention support system according to a third embodiment related to the present invention.
FIG. 14A is an explanatory diagram illustrating the infection spread prevention support system according to the third embodiment.
FIG. 14B is an explanatory diagram illustrating the infection spread prevention support system according to the third embodiment.
FIG. 14C is an explanatory diagram illustrating the infection spread prevention support system according to the third embodiment.
FIG. 14D is an explanatory diagram illustrating the infection spread prevention support system according to the third embodiment.
FIG. 14E is an explanatory diagram illustrating the infection spread prevention support system according to the third embodiment.
FIG. 15 is a conceptual diagram of a modification of the infection spread prevention support system according to the first embodiment.
FIG. 16 is a flow chart showing another example of the flow of the process of the infection spread prevention support system in the present embodiment.
FIG. 17 is a conceptual diagram of a modification of the infection spread prevention support system according to the second embodiment.
FIG. 18 is a conceptual diagram of a modification of the infection spread prevention support system according to the third embodiment.

### Description of Embodiments

### (First embodiment)

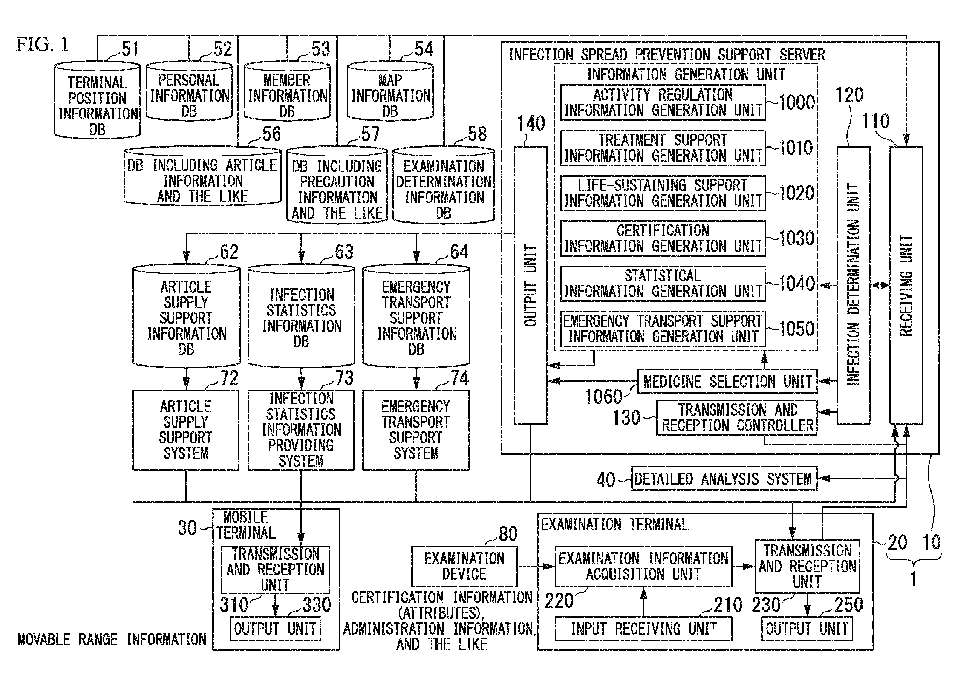

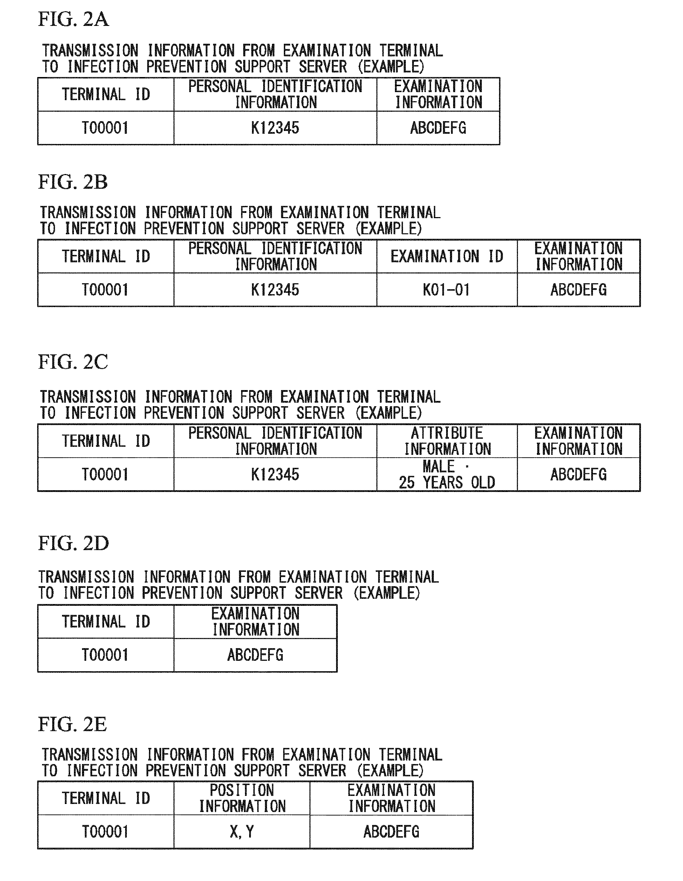

Hereinafter, a first embodiment of the present invention will be described referring to the drawings. FIG. 1 is a conceptual diagram of a system including an infection spread prevention support system according to the first embodiment of the present invention. FIGS. 2A to 8 are explanatory diagrams illustrating the infection spread prevention support system according to the first embodiment. As shown in FIG. 1, an infection spread prevention support system 1 according to the first embodiment of the present invention includes an infection spread prevention support server 10 and an examination terminal 20 that communicate with each other. The examination terminal 20 is a terminal for examining the infection of infectious diseases. The infection spread prevention support server 10 is a server for generating various kinds of information (will be described in detail later) based on information or the like transmitted from the examination terminal 20.

All of a terminal position information DB 51, a personal information DB 52, a member information DB 53, a map information DB 54, a DB including article information and the like 56, a DB including precaution information and the like 57, an examination determination information DB 58, an article supply support information DB 62, an infection statistics information DB 63, and an emergency transport support information DB 64 shown in FIG. 1 are external storage units (for example, databases) of the infection spread prevention support system 1 (will be described in detail later). In addition, all of a detailed analysis system 40, an article supply support system 72, an infection statistics information providing system 73, and an emergency transport support system 74 are external systems of the infection spread prevention support system 1 (will be described in detail later).

A mobile terminal 30 shown in FIG. 1 is an external terminal of the infection spread prevention support system 1. The mobile terminal 30 includes a transmission and reception unit (transmission unit and/or receiving unit) 310 and an output unit 330. In the mobile terminal 30, information is received by the transmission and reception unit 310, and the received information is output to the output unit 330. An example of the mobile terminal 30 is a mobile phone. However, the mobile terminal 30 does not necessarily need to have a call function, and an apparatus that outputs the received information (for example, a card type apparatus) may be used as described above.

The examination terminal 20 includes a mounting unit (not shown) for mounting an examination device 80, an input receiving unit 210, an examination information acquisition unit 220, a transmission and reception unit (transmission unit and/or receiving unit) 230, and an output unit 250. For example, the examination terminal 20 can perform examinations, such as genetic examination, microbiological examination, immunological examination, and biochemical examination, using the examination device 80.

The examination information acquisition unit 220 acquires examination information to examine the infection of infectious diseases. As an example, the examination information acquisition unit 220 acquires examination information from the examination device 80. When examination information is acquired, the examination information acquisition unit 220 supplies the examination information to the transmission and reception unit 230.

The input receiving unit 210 has an input interface for receiving input from the operator (for example, a subject) of the examination terminal 20. When there is input from the operator, the input receiving unit 210 supplies the input information to the transmission and reception unit 230. An example of the input information is personal identification information that identifies a subject, for example. The personal identification information may be directly input by the operator, or may be selected when the operator inputs a short code registered in advance so as to match the personal identification information, or may be information regarding the DNA of the subject obtained by the examination of the examination information acquisition unit 220. In addition, the examination information acquisition unit 220 may acquire examination information including the personal identification information that identifies a subject (for example, information regarding the DNA). In addition, the input of personal identification information is not necessary when the examination information acquisition unit 220 acquires examination information including the personal identification information that identifies a subject.

The transmission and reception unit 230 transmits various kinds of information to the infection spread prevention support server 10. As an example, when examination information is acquired from the examination information acquisition unit 220, the transmission and reception unit 230 transmits the examination information to the infection spread prevention support server 10. In addition, when the input information is acquired from the input receiving unit 210, the transmission and reception unit 230 transmits the input information to the infection spread prevention support server 10. In addition, the information transmitted from the examination terminal 20 to the infection spread prevention support server 10 differs depending on the input information or the like.

For example, the transmission and reception unit 230 transmits to the infection spread prevention support server 10 the transmission information including a terminal ID for identifying the examination terminal 20 (for example, zip code + alphanumeric characters of a predetermined number of digits), personal identification information, and examination information, which are shown in FIG. 2A. In addition, for example, the transmission and reception unit 230 may transmit to the infection spread prevention support server 10 the transmission information including an examination ID for identifying the examination content (type and level) in the system (FIG. 1), which is shown in FIG. 2B. In addition, for example, the transmission and reception unit 230 may transmit to the infection spread prevention support server 10 the transmission information including the attribute information (for example, sex, date of birth, blood type, medical history (personal and family), medicine administration history, family structure, and the like) of the subject shown in FIG. 2C. In addition, for example, when a user (subject) of the examination terminal 20 is specified as one person, the infection spread prevention support server 10 can identify the person from the terminal ID. Accordingly, the transmission and reception unit 230 may transmit the transmission information, which does not include the personal identification information shown in FIG. 2D, to the infection spread prevention support server 10. In addition, for example, when the location of use of the examination terminal 20 is not fixed, the location of use of the examination terminal 20 cannot be fixedly registered in the infection spread prevention support server 10. Accordingly, the transmission and reception unit 230 may transmit the transmission information including the position information of the examination terminal 20, which is shown in FIG. 2E, to the infection spread prevention support server 10.

In addition, the examination ID shown in FIG. 2B may be directly input by the operator, or may be automatically selected according to the shape of the examination device or the like. In addition, the attribute information shown in FIG. 2C may be directly input by the operator, or may be registered in the examination terminal 20 so as to match the personal identification information. In addition, the position information shown in FIG. 2E may be acquired using a GPS (in addition, in this case, the examination terminal 20 has a GPS function), or may be acquired by communication with an access point, such as a base station or a wireless LAN.

In addition, the transmission and reception unit 230 receives various kinds of information (will be described in detail later) from the infection spread prevention support server 10 and the like. When information is received from the infection spread prevention support server 10 and the like, the transmission and reception unit 230 supplies the received information to the output unit 250.

The output unit 250 outputs various kinds of information to the operator. For example, the output unit 250 displays an input screen, which is used for the input receiving unit 210 to acquire the input information, and the information received from the infection spread prevention support server 10 and the like on the output screen.

The infection spread prevention support server 10 includes a receiving unit 110, an infection determination unit 120, a transmission and reception controller 130, an activity regulation information generation unit 1000, a treatment support information generation unit 1010, a life-sustaining support information generation unit 1020, a certification information generation unit 1030, a statistical information generation unit 1040, an emergency transport support information generation unit 1050, a medicine selection unit 1060, and an output unit 140. In addition, in the following explanation, for the sake of convenience, the activity regulation information generation unit 1000, the treatment support information generation unit 1010, the life-sustaining support information generation unit 1020, the certification information generation unit 1030, the statistical information generation unit 1040, and the emergency transport support information generation unit 1050 are also referred to as an information generation unit, as shown by the dotted line.

The receiving unit 110 receives various kinds of information from the examination terminal 20. As an example, the receiving unit 110 selectively receives the information, which is transmitted from the examination terminal 20, according to the control information to selectively receive the information transmitted from the examination terminal 20 (hereinafter, referred to as selective reception control information). For example, the receiving unit 110 stores the selective reception control information temporarily and selectively receives the information transmitted from the examination terminal 20 based on the selective reception control information that is temporarily stored. In addition, the selective reception control information generated by the transmission and reception controller 130, and the details will be described later.

In addition, the receiving unit 110 acquires a detailed examination request (request for a detailed examination or determination to external instruments) including the examination information from the infection determination unit 120. When the detailed examination request is acquired, the receiving unit 110 outputs the detailed examination request to the detailed analysis system 40 and receives an analysis result or a determination result from the detailed analysis system 40 as a response to the detailed examination request.

In addition, the receiving unit 110 receives various kinds of information from external DBs. Hereinafter, external DBs will be described in detail.

Among the external DBs, the terminal position information DB 51, the personal information DB 52, the member information DB 53, the map information DB 54, the DB including article information and the like 56, the DB including precaution information and the like 57, and the examination determination information DB 58 mainly store the information provided to the infection spread prevention support server 10. Among the external DBs, the article supply support information DB 62, the infection statistics information DB 63, and the emergency transport support information DB 64 mainly store the information generated by the infection spread prevention support server 10.

The terminal position information DB 51 stores the position information of each terminal so as to match the terminal ID for identifying a terminal, such as the examination terminal 20 or the mobile terminal 30. In addition, as a method in which the terminal position information DB 51 stores the current position information, there are various methods. For example, for the mobile terminal 30 (the same for the examination terminal 20 which is a mobile terminal), the position information is appropriately updated in the same manner as in the location registration (location registration based on HLR (home location register)) of a mobile station, such as a mobile phone. In addition, as described above, the position information acquired by the mobile terminal 30 itself may be appropriately acquired by communication with a GPS or an access point and updated. In addition, for the examination terminal 20 (excluding the examination terminal 20 which is a mobile terminal), it is preferable to register the position information at the time of installation, for example.

The personal information DB 52 stores personal identification information, address, name, contact, emergency contact, attribute information (for example, sex, date of birth, blood type, medical history (personal and family), medicine administration history, family structure, and the like), authentication information (for example, a password or biometric information), a terminal ID of the examination terminal 20 used in the examination, and a terminal ID of the owned mobile terminal 30.

The member information DB 53 stores member identification information to identify a member, local information indicating the designated area that is designated by each member, and a terminal ID of the terminal designated by each member. The membership information DB 53 may store information (counting (output) item, frequency of generation, and generation time) regarding the infection statistics information (will be described later) designated by each member. In addition, members refer to public institutions (for example, national or local government), public institutions (hospitals, schools, trains, and buses), NPO, companies, and individuals, who are registered in advance.

The map information DB 54 stores map information (bitmap) linked with the position information (address information). That is, the map information DB 54 stores map information whose coordinates on the bitmap match the position information (longitude/latitude or address). In addition, the map information DB 54 stores a city code, a city name, and position information (address information) of each city. In addition, the map information DB 54 stores the position information (address information) of a predetermined location. Examples of a predetermined location are a fire department (department to which emergency services or a rescue team belong), a medical institution designated as a transport destination of an infected patient, a warehouse of articles (medicines, water, food, examination devices, and the like), a distribution station of articles, and the like.

The article information DB 56 stores information regarding the supply of articles, such as stock information of a predetermined article and a delivery company. Examples of a predetermined article are medicines, an examination device, a mask, a thermometer, water, food, and the like.

The DB including precaution information and the like 57 stores precaution statement and action guideline information indicating precaution statements or action guidelines offered to subjects or their families, information required to generate the activity plan information indicating the activity plan of the authorities to prevent the spread of infectious diseases, and the like. That is, materials (various forms and various kinds of wording corresponding to each column on each form) for automatically generating the information, such as the precaution statement and action guideline information and the activity plan information, are stored in the DB including precaution information and the like 57. In addition, the DB including precaution information and the like 57 supports various output forms (for example, foreign languages, audio, and braille) to meet foreigners who cannot understand Japanese and people with disabilities, and also stores information regarding the volunteers sent to people with mental disabilities and the like.
The examination determination information DB 58 stores information required to determine the presence or degree of infection of an infectious disease in the subject and the like. That is, examination determination information, such as a threshold value or a reference value for determining the presence or degree of infection of an infectious disease, for the examination information of the subject is stored in the examination determination information DB 58 in advance. For example, the examination determination information DB 58 stores a threshold value, which can determine the presence and degree of infection for a predetermined infectious disease, for each infectious disease. In addition, for example, the examination determination information DB 58 stores five steps of reference values (for example, reference values: 1 to 5), which can determine the presence and degree of infection for a predetermined infectious disease, for each infectious disease. As an example, data indicating "non-infected" when the reference value is 1 and 2, "infected" when the reference value is 3 or higher, and "serious illness" when the reference value is 5 is stored in advance in the examination determination information DB.

The article supply support information DB 62 stores the information generated by the information generation unit (mainly, the activity regulation information generation unit 1000, the treatment support information generation unit 1010, and the life-sustaining support information generation unit 1020). In addition, the article supply support information DB 62 is referred to by the article supply support system 72.

The infection statistics information DB 63 stores the information generated by the information generation unit (mainly, the statistical information generation unit 1040). The infection statistics information DB 63 is referred to by the infection statistics information providing system 73.

The emergency transport support information DB 64 stores the information generated by the information generation unit (mainly, the emergency transport support information generation unit 1050). The emergency transport support information DB 64 is referred to by the emergency transport support system 74.

Subsequently, an external system will be described. The article supply support system 72 is a system that provides the information stored in the article supply support information DB 62 to an apparatus (for example, a terminal) connected to the system. Therefore, for example, a dealer can check the information regarding the supply of an article (for example, delivery schedule) from an apparatus in the delivery orifice using the article supply support system 72. An apparatus connected to the article supply support system 72 may be limited to the member's apparatus.

The infection statistics information providing system 73 is a system that provides the information stored in the infection statistics information DB 63 to an apparatus connected to the system. Therefore, for example, a national or local government can check the infection statistics information from each apparatus using the infection statistics information providing system 73. An apparatus connected to the infection statistics information providing system 73 may be limited to the member's apparatus.

The emergency transport support system 74 is a system that provides the information stored in the emergency transport support information DB 64 to an apparatus connected to the system. Therefore, for example, a national or local government, NPO, and the like can check the emergency transport support information from each apparatus using the emergency transport support system 74. An apparatus connected to the emergency transport support system 74 may be limited to the member's apparatus.

Hereinafter, explanation regarding the receiving unit 110 of the infection spread prevention support server 10 is resumed. As described above, the receiving unit 110 receives various kinds of information from the external DB. For example, the receiving unit 110 receives information, which is required for the processing of the infection determination unit 120, the information generation unit, the medicine selection unit 1060, or the output unit 140 and which is not included in the information received from the examination terminal 20, by accessing the external DB. In addition, the receiving unit 110 may receive the information by accessing the external DB in response to the request from the external information generation unit, the medicine selection unit 1060, or the output unit 140, or may receive the information by accessing the external DB when the examination information is acquired from the examination terminal 20.

For example, when the information transmitted from the examination terminal 20 is transmission information (for example, transmission information shown in FIG. 2A) not including the position information of the examination terminal 20, the receiving unit 110 receives the position information (that is, position information of the examination terminal 20), which is matched with the terminal ID included in the transmission information, from the terminal position information DB 51. In addition, the position information of the examination terminal 20 (or the mobile terminal 30) is used as the position information of the subject.

The receiving unit 110 receives the information stored in the personal information DB 52, the terminal position information DB 51, the map information DB 54, and the like when the activity regulation information generation unit 1000 generates movement restriction information (will be described later).

The receiving unit 110 receives the information stored in the map information DB 54, the DB including article information and the like 56, and the like when the activity regulation information generation unit 1000 generates article supply support information to support the supply of articles used to prevent the spread of infection (for example, masks, protective clothing, and antiseptic solution), when the treatment support information generation unit 1010 generates article supply support information to support the supply of articles used in the treatment of infection (including medical-related articles that indirectly contribute to the treatment of infection (for example, medicines, an examination device, and a thermometer)), and when the life-sustaining support information generation unit 1020 generates article supply support information to support the supply of articles required for the maintenance of life of infected patients or their families (water and food).

The receiving unit 110 receives the information stored in the DB including precaution information and the like 57 when the treatment support information generation unit 1010 and the like generate the precaution statement and action guideline information and the activity plan information described above. In addition, the receiving unit 110 receives the information stored in the map information DB 54 and the like when the emergency transport support information generation unit 1050 generates emergency transport support information to support the transport of infected patients.

When the information generation unit (the activity regulation information generation unit 1000, the treatment support information generation unit 1010, the life-sustaining support information generation unit 1020, the certification information generation unit 1030, the statistical information generation unit 1040, and the emergency transport support information generation unit 1050) or the medicine selection unit 1060 generates output information using the attribute information of the subject (for example, when the statistical information generation unit 1040 generates infection statistics information for each attribute of the subject who is an infected patient, or when the medicine selection unit 1060 selects a medicine in consideration of the attributes of the subject who is an infected patient), the receiving unit 110 receives the attribute information, which is matched with the personal identification information included in the transmission information, from the personal information DB 52 if the information transmitted from the examination terminal 20 is transmission information (for example, transmission information shown in FIG. 2A) not including the attribute information of the subject.

The receiving unit 110 supplies to the infection determination unit 120 the information received from the examination terminal 20, the information received from the detailed analysis system 40, and the information received from the external DB. In addition, the terminal ID of the examination terminal 20 that the receiving unit 110 receives from the examination terminal 20 is an example of the information regarding the subject. In addition, the personal identification information of the subject, the attribute information of the subject, and the position information (position information of the subject) of the terminal (the examination terminal 20 and the mobile terminal 30) that the receiving unit 110 receives from the examination terminal 20 or the external DB are examples of the information regarding the subject.

The infection determination unit 120 acquires from the receiving unit 110 the information that the receiving unit 110 has received from the examination terminal 20, the information that the receiving unit 110 has received from the external DB, and the information that the receiving unit 110 has received from the detailed analysis system 40. The infection determination unit 120 determines the presence or degree of infection of an infectious disease using the examination information acquired from the receiving unit 110. In other words, the infection determination unit 120 determines that the subject is not infected with an infectious disease or determines the name of an infectious disease (including a suspected infectious disease)/symptom.

In addition, the infection determination unit 120 determines the presence or degree of infection of an infectious disease according to the predetermined examination content (type and level). As an example, when the examination ID is included in the information acquired from the receiving unit 110, the infection determination unit 120 determines the presence or degree of infection of an infectious disease according to the examination content identified by the examination ID. On the other hand, when no examination ID is included in the information acquired from the receiving unit 110, the infection determination unit 120 determines the presence or degree of infection of an infectious disease according to the examination content estimated from the examination information or the examination content set in advance. In addition, the infection determination unit 120 determines the presence or degree of infection of an infectious disease by comparing the examination information of the examination terminal 20 with the examination determination information stored in the storage unit (for example, the examination determination information DB 58). As an example, the infection determination unit 120 compares the examination information of the examination terminal 20 with a predetermined threshold value as the examination determination information stored in the examination determination information DB 58, and determines that the subject is infected with a predetermined infectious disease when the examination information is equal to or greater than the predetermined threshold value (infected) and determines that the subject is not infected with the predetermined infectious disease when the examination information is smaller than the predetermined threshold value (non-infected). In addition, for example, the infection determination unit 120 compares the examination information of the examination terminal 20 with five steps of predetermined reference values as the examination determination information stored in the examination determination information DB 58. The infection determination unit 120 determines that the subject is not infected with a predetermined infectious disease when the examination information is equivalent to the reference value 1 or 2, determines that the subject is infected with the predetermined infectious disease when the examination information is equivalent to the reference value 3 or higher, and determines that the symptom of an infectious disease is serious when the examination information is equivalent to the reference value 5.

In addition, the infection determination unit 120 may determine the presence or degree of infection of an infectious disease referring to the information (for example, sex, date of birth, medical history (personal and family), medicine administration history, family structure, and the like) that the receiving unit 110 has received from the personal information DB 52.

In addition, when determining the presence or degree of infection of an infectious disease, the infection determination unit 120 may transmit the above-described detailed examination request to the detailed analysis system 40 through the receiving unit 110 and receive the analysis result or the determination result from the detailed analysis system 40. When the analysis result or the determination result is received from the detailed analysis system 40, the infection determination unit 120 determines the presence or degree of infection of an infectious disease using the analysis result or the determination result. In addition, as determination regarding the presence or degree of infection of an infectious disease, the infection determination unit 120 may use the analysis result or the determination result received from the detailed analysis system 40 as it is.

When the presence or degree of infection of an infectious disease is determined, the infection determination unit 120 supplies the information that the receiving unit 110 has received from the examination terminal 20 (excluding the examination information), the information that the receiving unit 110 has received from the external DB, and the infection determination information which is a determination result of the infection determination unit 120 to the transmission and reception controller 130, the information generation unit (the activity regulation information generation unit 1000, the treatment support information generation unit 1010, the life-sustaining support information generation unit 1020, the certification information generation unit 1030, the statistical information generation unit 1040, and the emergency transport support information generation unit 1050), and the medicine selection unit 1060. In addition, the infection determination information is information indicating the presence or degree of infection of an infectious disease (determination that the subject is not infected with an infectious disease or the name of an infectious disease (including a suspected infectious disease))/symptom.

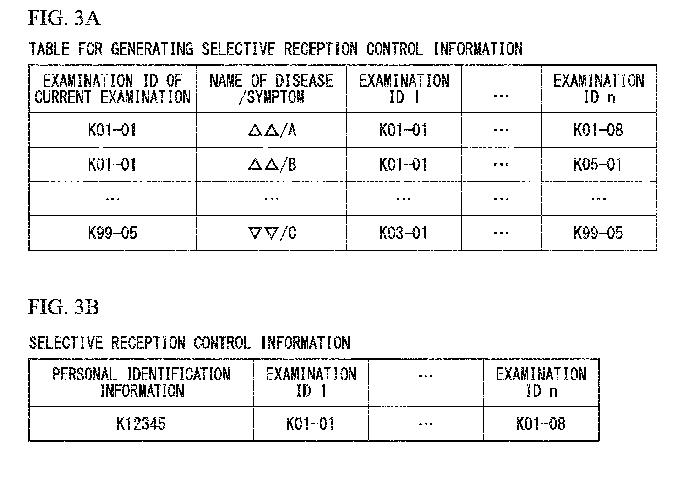

The transmission and reception controller 130 generates selective reception control information using the information acquired from the infection determination unit 120. For example, the transmission and reception controller 130 holds a table shown in FIG. 3A for generating selective reception control information, and generates the selective reception control information shown in FIG. 3B referring to the information (examination ID, personal identification information, the name of a disease, symptom) acquired from the infection determination unit 120 and the table for generating the selective reception control information. The table shown in FIG. 3A stores examination IDs of one or more examinations, which should be performed subsequently, so as to match the examination ID of the examination performed this time and the name of a disease/symptom. The selective reception control information shown in FIG. 3B includes personal identification information and examination IDs of one or more examinations that should be performed subsequently. When selective reception control information is generated, the transmission and reception controller 130 supplies the generated selective reception control information to the receiving unit 110. In addition, the receiving unit 110 selectively receives information in which the personal identification information and the examination ID match each other, of the information transmitted from the examination terminal 20, referring to the selective reception control information.

In addition, the transmission and reception controller 130 may generate selective reception control information including the terminal ID of the examination terminal 20 (that is, selective reception control information including the terminal ID of the examination terminal 20 and examination IDs of one or more examinations that should be performed subsequently) instead of the selective reception control information including the personal identification information. In addition, when the transmission and reception controller generates the selective reception control information including the terminal ID of the examination terminal 20, the receiving unit 110 selectively receives information in which the terminal ID of the examination terminal 20 and the examination ID match each other. In addition, the transmission and reception controller 130 may generate the selective reception control information including the terminal ID of the examination terminal 20 (for example, information not including the personal identification information) according to infection determination information (for example, the name of a disease or a symptom) and output the information selectively.

The medicine selection unit 1060 selects articles used in the treatment of infection (including medical-related articles that indirectly contribute to the treatment of infection; the same hereinbelow) using the information acquired from the infection determination unit 120. In addition, as described above, the "information acquired from the infection determination unit 120" by the medicine selection unit 1060 is information acquired from the examination terminal 20 (information regarding the subject), information acquired from the external DB (information regarding the subject and information other than the information regarding the subject, such as map information), and infection determination information. In addition, the information acquired from the external DB (information other than the information regarding the subject, such as map information) is obtained based on the information acquired from the examination terminal 20 (information regarding the subject) or the infection determination information. Therefore, "articles used in the treatment of infection are selected using the information acquired from the infection determination unit 120" is the same as "articles used in the treatment of infection are selected based on infection determination information and information regarding the subject".

For example, the medicine selection unit 1060 holds a table, which associates the name of a disease/symptom with medicines, and selects the types and quantities of articles (for example, medicines, an examination device, and a thermometer) used in the treatment of infection based on the table, the infection determination information, the information of the personal information DB 52 (for example, medical history (personal and family) and medicine administration history), and the like.

Moreover, in addition to the articles used in the treatment of infection, the medicine selection unit 1060 selects the types and quantities of articles used to prevent the spread of infection (for example, masks, protective clothing, and antiseptic solution) and the types and quantities of articles required to maintain the lives of infected patients or their families (for example, water and food).

The medicine selection unit 1060 outputs the article selection information including the types and quantities of selected articles to the information generation unit (the activity regulation information generation unit 1000, the treatment support information generation unit 1010, the life-sustaining support information generation unit 1020, the certification information generation unit 1030, the statistical information generation unit 1040, and the emergency transport support information generation unit 1050) or the output unit 140.

The information generation unit (the activity regulation information generation unit 1000, the treatment support information generation unit 1010, the life-sustaining support information generation unit 1020, the certification information generation unit 1030, the statistical information generation unit 1040, and the emergency transport support information generation unit 1050) generates various kinds of information using the information acquired from the infection determination unit 120 or the medicine selection unit 1060.

In addition, the "information acquired from the medicine selection unit 1060" is originally information acquired based on the "information acquired from the infection determination unit 120". Therefore, "various kinds of information are generated using the information acquired from the infection determination unit 120 or the medicine selection unit 1060" is the same as "various kinds of information are generated based on infection determination information and information regarding the subject".

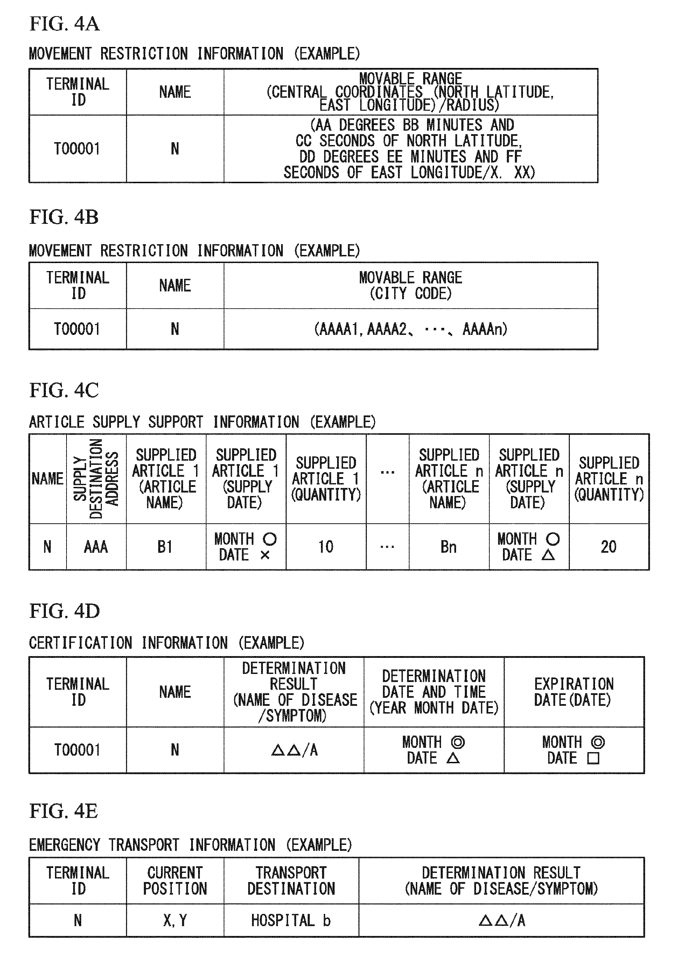

The activity regulation information generation unit 1000 specifies infected patients of the infectious disease among the subjects based on the infection determination information and the information regarding the subject and generates activity regulation information to regulate the activities of the specified infected patients. For example, the activity regulation information generation unit 1000 generates movement restriction information, which indicates a movable range of an infected patient, as the activity regulation information. In addition, the activity regulation information generation unit 1000 generates movement restriction information including the terminal ID of a transmission destination terminal to which the movement restriction information is transmitted, the name of the subject determined to be an infected patient, and a movable range defined by the central coordinates and the radius, for example, as shown in FIG. 4A.

In addition, the "terminal ID of the transmission destination terminal" included in the movement restriction information in FIG. 4A is the terminal ID (information regarding the subject) of the examination terminal 20, which is a transmission source of examination information, and/or the terminal ID (information regarding the subject) of the mobile terminal 30 owned by the subject determined to be the infected patient.

In addition, the activity regulation information generation unit 1000 determines the "movable range" included in the movement restriction information in FIG. 4A based on the name of a disease/symptom (infection determination information) and the position information (information regarding the subject) of the subject which is the position information of the examination terminal 20 or the mobile terminal 30. For example, the activity regulation information generation unit 1000 holds a table showing the movable distance for each name of a disease/symptom, and determines a movable range based on the table, infection determination information, position information of the subject, information of the map information DB 54, and the like.

In addition, the activity regulation information generation unit 1000 may generate movement restriction information including the terminal ID of a transmission destination terminal to which the movement restriction information is transmitted, the name of the subject determined to be an infected patient, and a movable range defined by the city code as shown in FIG. 4B, instead of or in addition to the movement restriction information shown in FIG. 4A.

In addition, the activity regulation information generation unit 1000 generates precaution statement and action guideline information, which indicates precaution statements or action guidelines offered to subjects or their families, as the activity regulation information. For example, as described above, the activity regulation information generation unit 1000 determines a movable range from the name of a disease/symptom (infection determination information) and the position information of the subject (information regarding the subject), and generates message information indicating that going out beyond the range is forbidden.

In addition, the activity regulation information generation unit 1000 generates activity plan information, which indicates the activity plan of the authorities to prevent the spread of infectious diseases, as the activity regulation information. For example, the activity regulation information generation unit 1000 generates activity plan information based on infection determination information of a plurality of subjects, information of the DB including precaution information and the like 57, and the like.

In addition, the activity regulation information generation unit 1000 generates article supply support information to support the supply of articles used to prevent the spread of infection (for example, masks, protective clothing, and antiseptic solution) based on the infection determination information and the information regarding the subject. For example, the activity regulation information generation unit 1000 generates article supply support information based on the infection determination information, the position information of the subject, the information of the map information DB 54, and the information of the DB including article information and the like 56, and the like. For example, the activity regulation information generation unit 1000 generates article supply support information including the name and address of the subject determined to be an infected patient, the address of the supply destination, names of supplied articles, and supply date, as shown in FIG. 4C. In addition, the activity regulation information generation unit 1000 determines supplied articles based on the information (article selection information) acquired from the medicine selection unit 1060. In addition, from the viewpoint of preventing the spread of infectious diseases, it is preferable that the activity regulation information generation unit 1000 determine the supply destination of articles used to prevent the spread of infection.

In addition, the activity regulation information generation unit 1000 generates activity regulation lifting information to lift the activity regulation of an infected patient based on the infection determination information and the information regarding the subject. For example, the activity regulation information generation unit 1000 generates message information, which indicates that the limit of the movable range has been lifted and which is to be transmitted to the terminal that has transmitted the movement restriction information shown in FIGS. 4A and 4B. In addition, for example, the activity regulation information generation unit 1000 generates movement restriction information indicating an infinite movable range.

In addition, the activity regulation information generation unit 1000 may generate activity regulation information according to the attributes of the subject based on the infection determination information and the information regarding the subject including the attribute information of the subject. For example, the activity regulation information generation unit 1000 holds a table showing the movable distance for each name of a disease/symptom and each attribute of the subject, and generates movement restriction information based on the table, infection determination information, position information of the subject, information of the map information DB 54, and the like. Similarly, the activity regulation information generation unit 1000 may generate precaution statement and action guideline information, activity plan information, and article supply support information according to the attributes of the subject.

The treatment support information generation unit 1010 specifies infected patients of the infectious disease among the subjects based on the infection determination information and the information regarding the subject and generates treatment support information to support the treatment of the specified infectious diseases. For example, the treatment support information generation unit 1010 generates article supply support information to support the supply of articles used in the treatment of infection (including medical-related articles that indirectly contribute to the treatment of infection (for example, medicines, an examination device, and a thermometer)) as the treatment support information. The method used when the treatment support information generation unit 1010 generates the article supply support information to support the supply of articles used in the treatment of infection is the same as the method used when the activity regulation information generation unit 1000 generates the article supply support information to support the supply of articles used to prevent the spread of infection. In addition, also from the viewpoint of preventing the spread of infectious diseases, it is preferable that the treatment support information generation unit 1010 determine the supply destination of articles used in the treatment of infection.

In addition, as the treatment support information, the treatment support information generation unit 1010 generates precaution statement and action guideline information indicating precaution statements or action guidelines offered to infected patients or their families and activity plan information indicating the activity plan of the authorities to treat infectious diseases. In addition, the method used when the treatment support information generation unit 1010 generates the precaution statement and action guideline information and the activity plan information is the same as the method used when the activity regulation information generation unit 1000 generates the precaution statement and action guideline information and the activity plan information.

In addition, similar to the activity regulation information generation unit 1000, the treatment support information generation unit 1010 may generate treatment support information according to the attributes of the subject based on the infection determination information and the information regarding the subject including the attribute information of the subject.

The life-sustaining support information generation unit 1020 specifies infected patients of the infectious disease among the subjects based on the infection determination information and the information regarding the subject and generates life-sustaining support information to support the maintenance of the lives of the specified infected patients. For example, the life-sustaining support information generation unit 1020 generates article supply support information to support the supply of articles (for example, water and food), which are required to maintain the lives of infected patients, as the life-sustaining support information. The method used when the life-sustaining support information generation unit 1020 generates the article supply support information to support the supply of articles required to maintain the lives of infected patients or their families is the same as the method used when the activity regulation information generation unit 1000 generates the article supply support information to support the supply of articles used to prevent the spread of infection. In addition, also from the viewpoint of preventing the spread of infectious diseases, it is preferable that the life-sustaining support information generation unit 1020 determine the supply destination of articles required to maintain the lives of infected patients.

In addition, as the life-sustaining support information, the life-sustaining support information generation unit 1020 generates precaution statement and action guideline information indicating precaution statements or action guidelines offered to infected patients or their families and activity plan information indicating the activity plan of the authorities to treat infectious diseases. In addition, the method used when the life-sustaining support information generation unit 1020 generates the precaution statement and action guideline information and the activity plan information is the same as the method used when the activity regulation information generation unit 1000 generates the precaution statement and action guideline information and the activity plan information.

In addition, similar to the activity regulation information generation unit 1000, the life-sustaining support information generation unit 1020 may generate life-sustaining support information according to the attributes of the subject based on the infection determination information and the information regarding the subject including the attribute information of the subject.

The certification information generation unit 1030 generates certification information including a determination result (infection determination information) and determination date and time based on the infection determination information and the information regarding the subject. For example, the certification information generation unit 1030 generates certification information including the terminal ID of a transmission destination terminal to which the certification information is transmitted, the name of the subject determined to be an infected patient, a determination result, determination date and time, and expiration date, as shown in FIG. 4D. In addition, the expiration date included in the certification information is determined based on the determination result (the name of a disease/symptom).

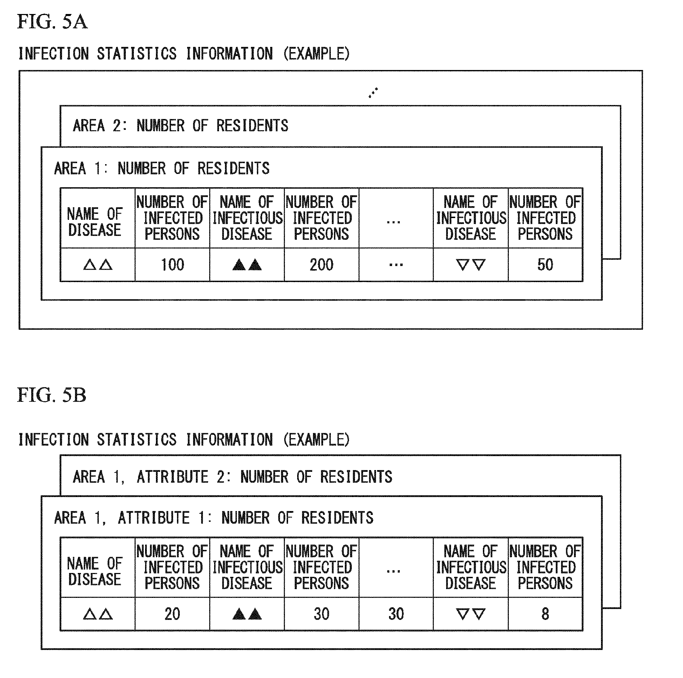

The statistical information generation unit 1040 specifies infected patients of the infectious disease among the subjects based on the infection determination information and the information regarding the subject and generates infection statistics information. For example, the statistical information generation unit 1040 generates infection statistics information, which indicates the total number of infected patients for each name of a disease, for each area, as shown in FIG. 5A. In addition, for example, the statistical information generation unit 1040 may generate infection statistics information, which indicates the total number of infected patients for each name of a disease, for each area and each attribute of the subject who is an infected patient, as shown in FIG. 5B. In addition, for example, the statistical information generation unit 1040 may generate infection statistics information (not shown), which indicates the total number of infected patients for each name of a disease and each symptom, instead of the infection statistics information indicating the total number of infected patients for each name of a disease. In addition, for example, when the infection statistics information is provided to a member, the statistical information generation unit 1040 may change the counting items for each member to generate infection statistics information (not shown), or may change the frequency of generation and generation time for each member to generate infection statistics information.

The emergency transport support information generation unit 1050 specifies infected patients of the infectious disease among the subjects based on the infection determination information and the information regarding the subject and generates emergency transport support information to support the transport of the specified infected patients. For example, the emergency transport support information generation unit 1050 generates emergency transport support information including the name of the subject determined to be an infected patient, current position, medical institution as transport destination, and a determination result, as shown in FIG. 4E. in addition, the transport destination included in the emergency transport support information is determined based on the infection determination information, the position information of the subject, the information of the map information DB 54, and the like.

The information generation unit (the activity regulation information generation unit 1000, the treatment support information generation unit 1010, the life-sustaining support information generation unit 1020, the certification information generation unit 1030, the statistical information generation unit 1040, and the emergency transport support information generation unit 1050) supplies the information acquired from the infection determination unit 120 and each item of the generated information to the output unit 140.

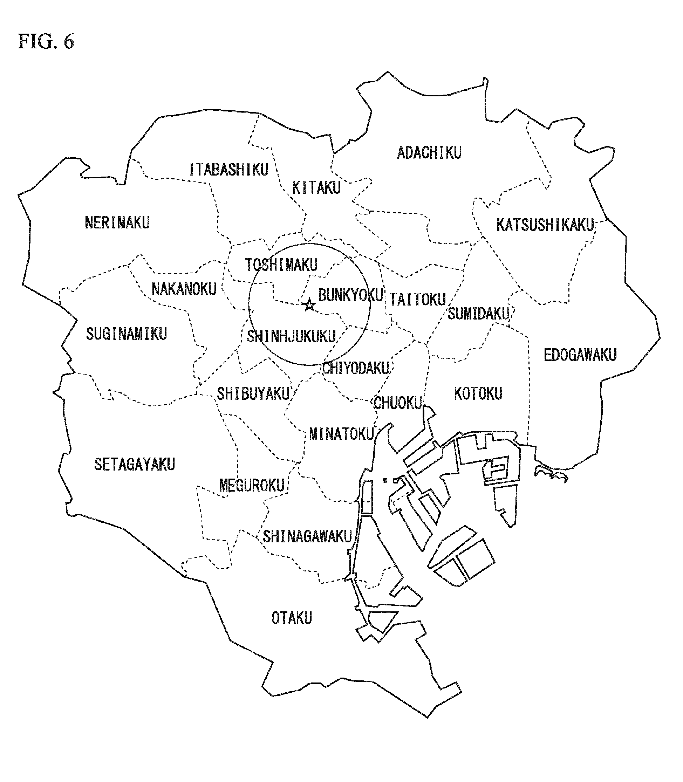

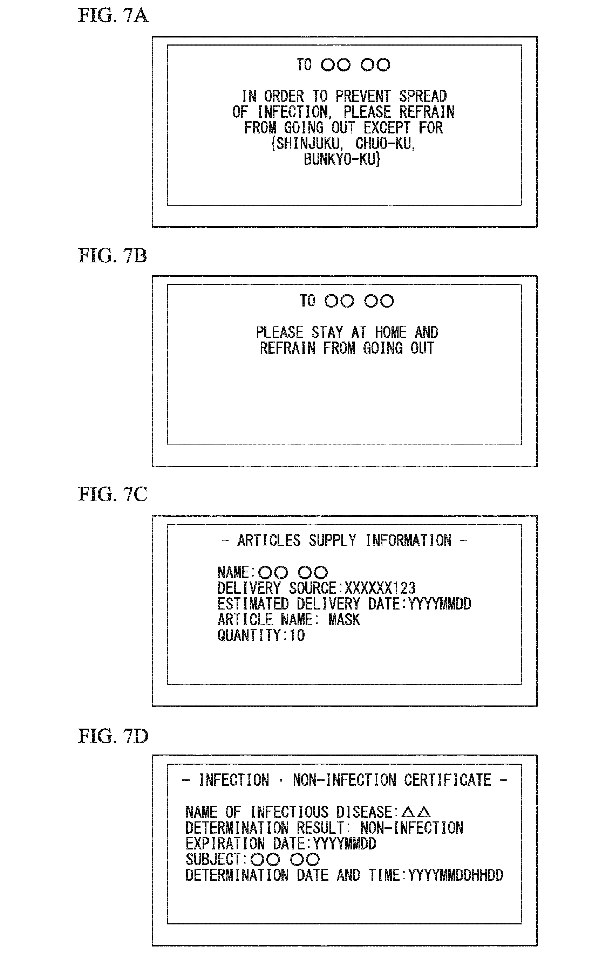

When the movement restriction information is acquired from the activity regulation information generation unit 1000, the output unit 140 transmits the movement restriction information to the transmission destination terminal (the examination terminal 20 and the mobile terminal 30). In addition, the terminal that receives the movement restriction information displays information indicating a movable range based on the movement restriction information on the output unit 250. For example, the examination terminal 20 (mobile terminal 30) displays information indicating a movable range shown in FIG. 6 on the output unit 250 (output unit 330) when the movement restriction information shown in FIG. 4A is received. In addition, in FIG. 6, the asterisk indicates a current position of the subject determined to be an infected patient, and the circle surrounding the asterisk indicates a movable range. In addition, for example, the examination terminal 20 (mobile terminal 30) displays information indicating a movable range shown in FIG. 7A on the output unit 250 (output unit 330) when the movement restriction information shown in FIG. 4B is received.

In addition, based on member information which is the member information regarding members to whom the activity regulation information is provided and which includes a designated area and a designated output destination that are designated by each member, the output unit 140 may output the activity regulation information of the subject in the designated area to the designated terminal (not shown) of the member. In addition, when information (for example, name) allowing the subject to be identified is included in the information transmitted to the designated terminal of the member (for example, the movement control information in FIG. 2A), the output unit 140 may cipher (for example, delete) the information allowing the subject to be identified and transmit the information to the terminal (not shown) of the member.

In addition, when the precaution statement and action guideline information is acquired from the activity regulation information generation unit 1000 (the same for the treatment support information generation unit 1010 and the life-sustaining support information generation unit 1020), the output unit 140 transmits the precaution statement and action guideline information to the transmission destination terminal (the examination terminal 20 and the mobile terminal 30). In addition, the terminal that receives the precaution statement and action guideline information displays the precaution statement and action guideline information on the output unit 250 (output unit 330). For example, the examination terminal 20 (mobile terminal 30) displays a precaution statement shown in FIG. 7B on the output unit 250 (output unit 330). In addition, when the activity regulation information generation unit 1000 generates precaution statement and action guideline information for foreigners who cannot understand Japanese and people with disabilities (for example, foreign languages, audio, and braille), the examination terminal 20 (mobile terminal 30) displays the precaution statement and action guideline information for foreigners who cannot understand Japanese and people with disabilities. When the activity plan information is acquired from the activity regulation information generation unit 1000, the output unit 140 transmits the activity plan information to the terminal (not shown) of the authorities.

In addition, when the article supply support information is acquired from the activity regulation information generation unit 1000 (the same for the treatment support information generation unit 1010 and the life-sustaining support information generation unit 1020), the output unit 140 stores (registers) the article supply support information in the article supply support information DB 62. In addition, the article supply support system 72 outputs the article supply support information stored in the article supply support information DB 62 to the terminal of the user of the article supply support system 72. In addition, the terminal of the user displays the article supply support information as shown in FIG. 7C, for example. In addition, when the use of the article supply support system 72 is limited to members (for example, authorities who control the supply of articles, such as medicines, water, and food, and dealers relevant to the supply of the articles), the use of article supply support information is limited to members. However, the method of limiting the use of article supply support information to members is not limited to this. For example, based on member information which is the member information regarding members to whom the article supply support information is provided and which includes a designated area and a designated output destination that are designated by each member, the output unit 140 may output the article supply support information of the subject in the designated area to the designated terminal (not shown) of the member.

In addition, when the activity regulation lifting information is acquired from the activity regulation information generation unit 1000, the output unit 140 transmits the activity regulation lifting information to the transmission destination terminal (the examination terminal 20 and the mobile terminal 30).

In addition, when the certification information is acquired from the certification information generation unit 1030, the output unit 140 transmits the certification information to the transmission destination terminal (the examination terminal 20 and the mobile terminal 30). In addition, the terminal that receives the certification information displays the certification information on the output unit 250 (output unit 330). For example, the examination terminal 20 (mobile terminal 30) displays the certification information on the output unit 250 (output unit 330) as shown in FIG. 7D. In addition, the output unit 140 may transmit the certification information to a terminal (not shown) of the authorities.

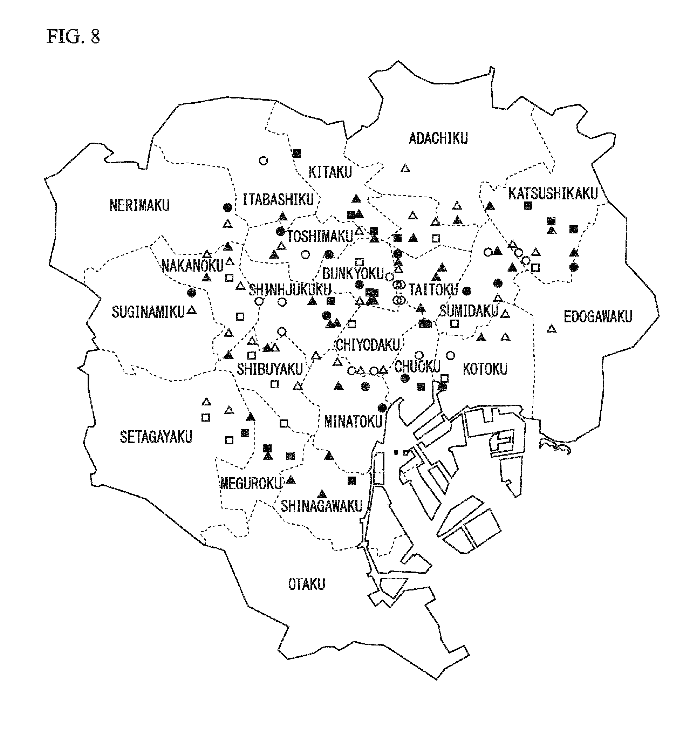

In addition, when the infection statistics information is acquired from the statistical information generation unit 1040, the output unit 140 registers (stores) the infection statistics information in the infection statistics information DB 63. In addition, the infection statistics information providing system 73 outputs the infection statistics information stored in the infection statistics information DB 63 to the terminal of the user of the infection statistics information providing system 73. In addition, for example, the terminal of the user may display the infection statistics information, or may display the distribution of infected patients based on the infection statistics information as shown in FIG. 8. FIG. 8 shows the distribution of infected patients according to the attributes (circles, squares, and triangles) and the symptoms (black marks and white marks) of infected patients for a certain infectious disease. In addition, when the use of the infection statistics information providing system 73 is limited to members (for example, authorities and research firms who use statistical information), the use of infection statistics information is limited to members. However, the method of limiting the use of infection statistics information to members is not limited to this. For example, based on member information which is the member information regarding members to whom the infection statistics information is provided and which includes a designated area and a designated output destination that are designated by each member, the output unit 140 may output the infection statistics information counted for the designated area to the designated terminal (not shown) of the member.

In addition, when the emergency transport support information is acquired from the emergency transport support information generation unit 1050, the output unit 140 registers (stores) the emergency transport support information in the emergency transport support information DB 64. In addition, the emergency transport support system 74 outputs the emergency transport support information stored in the emergency transport support information DB 64 to the terminal of the user of the emergency transport support system 74. In addition, when the use of the emergency transport support system 74 is limited to members (for example, authorities and consignment company), the use of emergency transport support information is limited to members. However, the method of limiting the use of emergency transport support information to members is not limited to this. For example, based on member information which is the member information regarding members to whom the emergency transport support information is provided and which includes a designated area and a designated output destination that are designated by each member, the output unit 140 may output the emergency transport support information in the designated area to the designated terminal (not shown) of the member.

In addition, when the article selection information is acquired from the medicine selection unit 1060, the output unit 140 transmits the article selection information and the certification information to the transmission destination terminal (the examination terminal 20 and the mobile terminal 30). In addition, the output unit 140 may transmit the article selection information to the designated terminal (not shown) of a member (for example, the authorities who control the supply of articles, such as medicines, water, and food, and dealers relevant to the supply of the articles) based on the member information. In addition, the output unit 140 may transmit the article selection information relevant to the area designated by the member.

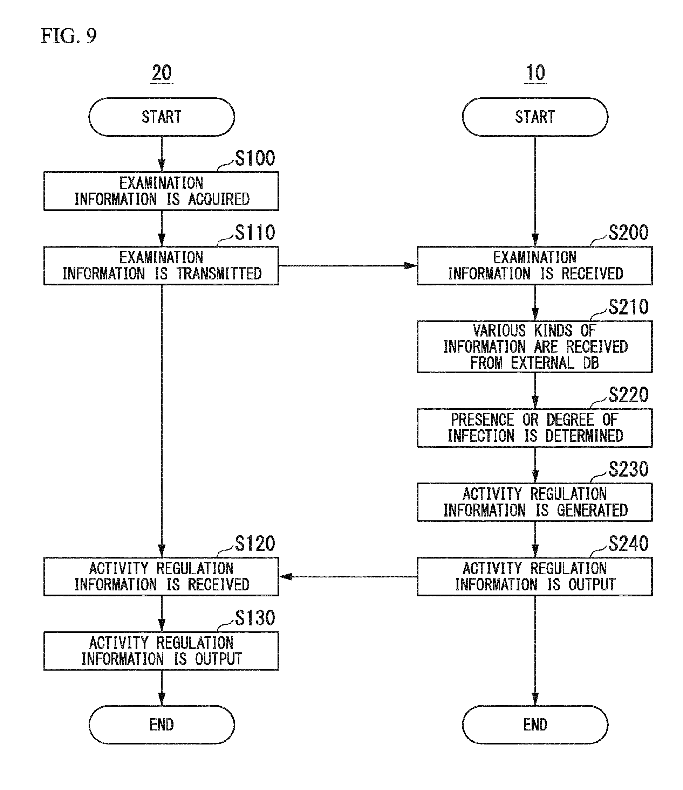

Hereinafter, the flow of the process of the infection spread prevention support system 1 will be described referring to FIG. 9. In addition, the flow chart shown in FIG. 9 is an example in which, in the configuration shown in FIG. 1, the infection spread prevention support server 10 generates activity regulation information and transmits the generated activity regulation information to the examination terminal 20. In addition, in FIG. 9, the left side shows the process of the examination terminal 20, and the right side shows the process of the infection spread prevention support server 10.

The examination information acquisition unit 220 of the examination terminal 20 acquires examination information to examine the infection of infectious diseases (step S100). The transmission and reception unit 230 of the examination terminal 20 transmits the examination information to the infection spread prevention support server 10 (step S 110). In addition, when the input information is acquired from the input receiving unit 210, the transmission and reception unit 230 of the examination terminal 20 transmits the input information and the examination information to the infection spread prevention support server 10.

The receiving unit 110 of the infection spread prevention support server 10 receives the information transmitted from the examination terminal 20 (step S200). The receiving unit 110 of the infection spread prevention support server 10 receives information, which is required to generate activity regulation information, from the external DB (step S210). The infection determination unit 120 of the infection spread prevention support server 10 determines the presence or degree of infection of an infectious disease (step S220). The activity regulation information generation unit 1000 of the infection spread prevention support server 10 generates activity regulation information to regulate the activities of infected patients based on the infection determination information and the information regarding the subject (step S230). The output unit 140 of the infection spread prevention support server 10 transmits the activity regulation information to the examination terminal 20 (step S240).

The transmission and reception unit 230 of the examination terminal 20 receives the activity regulation information transmitted from the infection spread prevention support server 10 (step S120). The output unit 250 of the examination terminal 20 displays the activity regulation information (step S130). Then, this flow chart is ended.

As described above, according to the first embodiment, it is possible to support the prevention of the spread of infectious diseases. That is, according to the first embodiment, the spread of infectious diseases can be prevented by regulating the activities of infected patients using the activity regulation information or by prompting the treatment of infectious diseases using the treatment support information. In addition, according to the present invention, from the viewpoint of preventing the spread of infectious diseases, the spread of infectious diseases can be prevented by controlling the supply destination of articles required to maintain the lives of infected patients.

In addition, as a mode to control the transmission and reception of information between the infection spread prevention support server 10 and the examination terminal 20, the mode has been described in which the transmission and reception controller 130 of the infection spread prevention support server 10 generates selective reception control information, which is required when the receiving unit 110 selectively receives the information transmitted from the examination terminal 20, using the information acquired from the infection determination unit 120. However, the mode to control the transmission and reception of information between the infection spread prevention support server 10 and the examination terminal 20 is not limited to this. For example, the transmission and reception controller 130 may generate selective transmission control information (having the same content as the selective reception control information), which is required when the examination terminal 20 selectively transmits the information received by the infection spread prevention support server 10 (that is, information transmitted to the infection spread prevention support server 10), using the information acquired from the infection determination unit 120. In the mode in which the transmission and reception controller 130 generates selective transmission control information, the transmission and reception controller 130 transmits the selective transmission control information to the examination terminal 20 through the output unit 140. The transmission and reception unit 230 of the examination terminal 20 that receives the selective transmission control information refers to the selective transmission control information and selectively transmits information in which the personal identification information and the examination ID match each other, of the information acquired from the examination information acquisition unit 220, to the infection spread prevention support server 10.

In addition, in the above explanation, the mode has been described in which the examination information acquisition unit 220 of the examination terminal 20 acquires the examination information including the personal identification information to identify the subject (for example, information regarding DNA). In the mode, however, the infection determination unit 120 of the infection spread prevention support server 10 may perform the subject identification process based on the personal identification information of the subject stored in the personal information DB 52 in advance (information regarding DNA that is used as biometric information) and the personal identification information acquired from the examination information received from the examination terminal 20 and determine the presence or degree of infection when the subject is authenticated. In addition, the infection determination unit 120 of the infection spread prevention support server 10 may also perform the subject identification process based on the password of the personal identification information of the subject, which is stored in the personal information DB 52 in advance, and the password received from the examination terminal 20 (password received by the input receiving unit 210).

In addition, in the above explanation, the case has been described in which the examination terminal 20 includes a mounting unit for mounting the examination device 80 and the examination information is acquired from the examination device 80. However, the examination terminal 20 may acquire examination information, which is acquired by other apparatuses, through cable or wireless communication or media, such as a USB memory.

In addition, in the first embodiment, the configuration has been described in which the terminal position information DB 51, the personal information DB 52, the member information DB 53, the map information DB 54, the DB including article information and the like 56, the DB including precaution information and the like 57, the examination determination information DB 58, the article supply support information DB 62, the infection statistics information DB 63, and the emergency transport support information DB 64 are present outside the infection spread prevention support server 10. However, a configuration may be adopted in which the infection spread prevention support server 10 includes one or two or more of the above-described DBs (the same for infection spread prevention support servers in other embodiments that will be described later). In addition, in the first embodiment, the configuration has been described in which the detailed analysis system 40, the article supply support system 72, the infection statistics information providing system 73, and the emergency transport support system 74 are present outside the infection spread prevention support server 10. However, a configuration may be adopted in which the infection spread prevention support server 10 includes one or two or more of the above-described systems (the same for infection spread prevention support servers in other embodiments that will be described later).

### (Second embodiment)

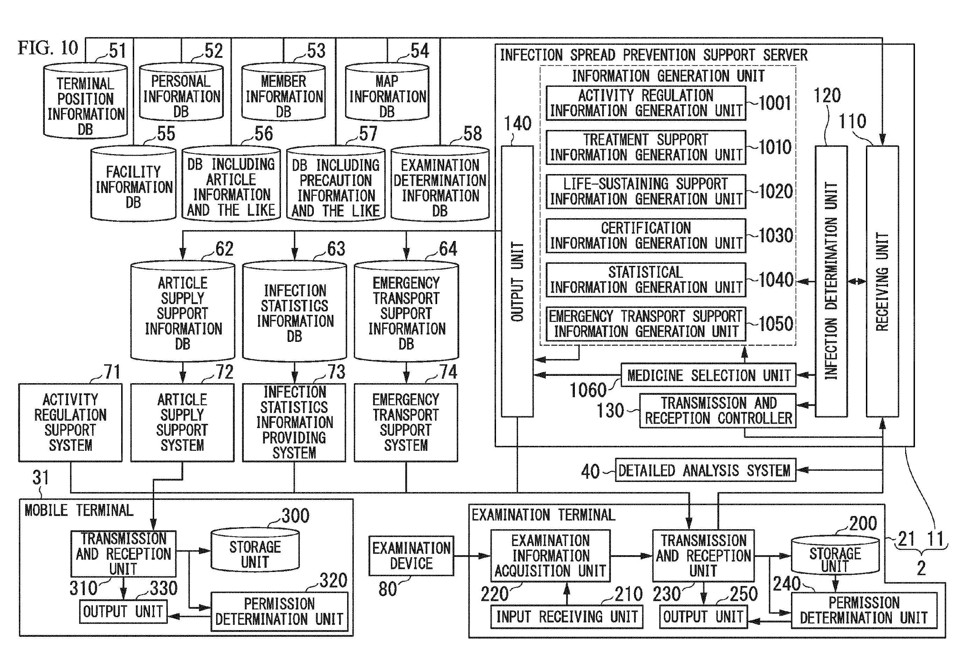

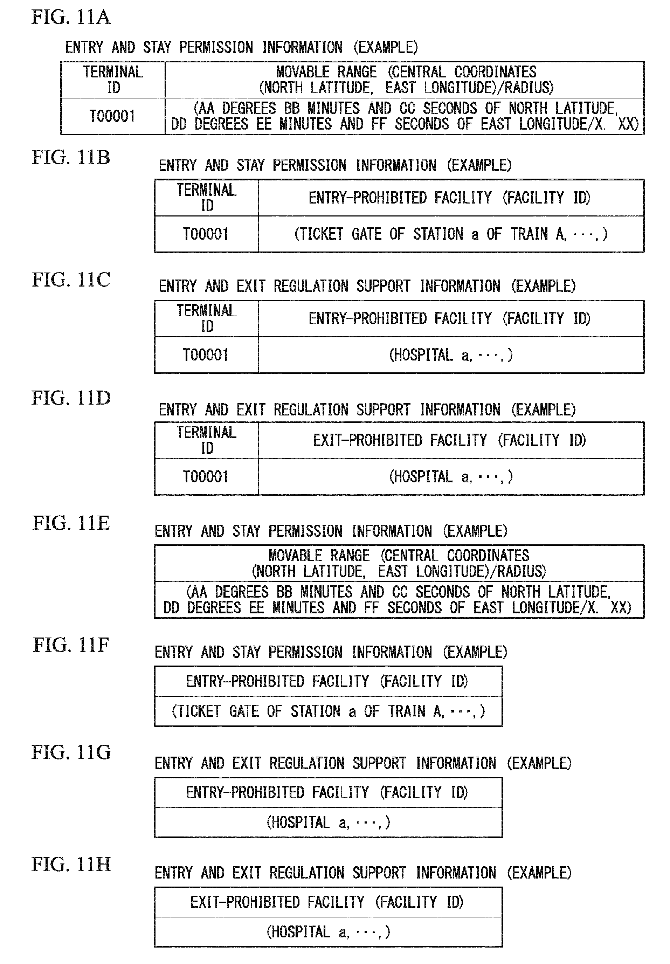

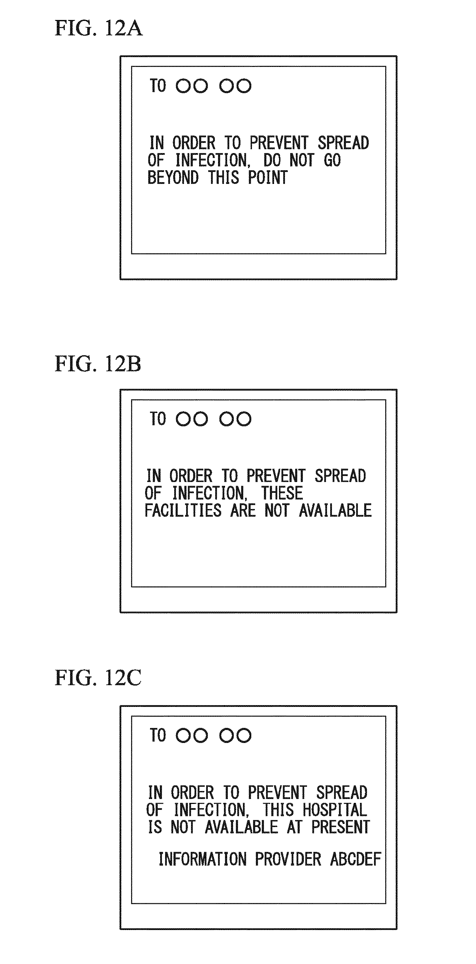

Hereinafter, a second embodiment of the present invention will be described referring to the drawings. FIG. 10 is a conceptual diagram of a system including an infection spread prevention support system according to the second embodiment of the present invention. FIGS. 11A to 11H and FIGS. 12A to 12C are explanatory diagrams illustrating the infection spread prevention support system according to the second embodiment. As shown in FIG. 10, an infection spread prevention support system 2 according to the second embodiment is configured to include an infection spread prevention support server 11 and an examination terminal 21 that communicate with each other.

All of a terminal position information DB 51, a personal information DB 52, a member information DB 53, a map information DB 54, a facility information DB 55, a DB including article information and the like 56, a DB including precaution information and the like 57, an examination determination information DB 58, an article supply support information DB 62, an infection statistics information DB 63, and an emergency transport support information DB 64 shown in FIG. 10 are external DBs of the infection spread prevention support system 2. In addition, all of a detailed analysis system 40, an activity regulation support system 71, an article supply support system 72, an infection statistics information providing system 73, and an emergency transport support system 74 are external DBs of the infection spread prevention support system 2. In addition, external DBs other than the facility information DB 55 and external systems other than the activity regulation support system 71 are the same as those described in the first embodiment of the present invention.

A mobile terminal 31 shown in FIG. 10 is an external terminal of the infection spread prevention support system 2. Similar to the mobile terminal 30 described in the first embodiment of the present invention, an example of the mobile terminal 31 does not necessarily need to have a call function, and an apparatus that outputs the received information (for example, a card type apparatus) may be used as described above.

The mobile terminal 31 includes a storage unit 300, a transmission and reception unit 310, a permission determination unit 320, and an output unit 330. The mobile terminal 31 is different from the mobile terminal 30 described in the first embodiment in that the mobile terminal 31 includes the storage unit 300 and the permission determination unit 320. In addition, the output unit 330 of the mobile terminal 31 has a display function, an audio output function, a lighting function, a vibration function, and the like.

The facility information DB 55 mainly stores information provided to the infection spread prevention support server 11. As an example, the facility information DB 55 stores position information and identification information (facility ID) of a predetermined facility. An example of the predetermined facility is a door automatically opened and closed by electronic control (for example, an automatic door of the entrance of a building), a gate (for example, an automatic ticket gate installed by the passenger company), and a monitoring camera automatically controlled by electronic control.

The activity regulation support system 71 is a system that supports the regulation of the passage of people or entry and exit of people or supports the monitoring of people. The activity regulation support system 71 stores the facility ID of a predetermined facility for regulating the passage of people or entry and exit and also controls the driving of the predetermined facility. In addition, the activity regulation support system 71 stores the facility ID of a predetermined facility for monitoring people and also controls the driving of the predetermined facility. An example of the predetermined facility is a door automatically opened and closed by electronic control (for example, an automatic door of the entrance of a building), a gate (for example, an automatic ticket gate installed by the passenger company), and a monitoring camera automatically controlled by electronic control.

The infection spread prevention support server 11 is different from the infection spread prevention support server 10 according to the first embodiment in that the infection spread prevention support server 11 includes an activity regulation information generation unit 1001 instead of the activity regulation information generation unit 1000. Therefore, some or all of the explanation regarding units in common with the infection spread prevention support server 10 will be omitted.

The activity regulation information generation unit 1001 specifies infected patients of the infectious disease among the subjects based on infection determination information and information regarding the subject and generates activity regulation information to regulate the activities of the specified infected patients. For example, as the activity regulation information, the activity regulation information generation unit 1001 generates entry and stay regulation information to regulate the entry or stay of infected patients or entry and exit regulation support information to regulate the entry and exit to and from the medical institution for the subjects.

In addition, the activity regulation information generation unit 1001 generates entry and stay regulation information including the terminal ID of a transmission destination terminal, to which the entry and stay regulation information is transmitted, and a movable range defined by the central coordinates and the radius, for example, as shown in FIG. 11A. In addition, the "terminal ID of the transmission destination terminal" included in the entry and stay regulation information in FIG. 11A is the terminal ID (information regarding the subject) of the examination terminal 21, which is a transmission source of examination information, and/or the terminal ID (information regarding the subject) of the mobile terminal 31 owned by the subject determined to be the infected patient. In addition, the "movable range" included in the entry and stay regulation information in FIG. 11A is determined based on the name of a disease/symptom (infection determination information) and the position information (information regarding the subject) of the subject which is the position information of the examination terminal 21 or the mobile terminal 31.

In addition, for example, the activity regulation information generation unit 1001 generates entry and stay regulation information including the terminal ID of a transmission destination terminal, to which the entry and stay regulation information is transmitted, and the facility ID of an entry-prohibited facility as shown in FIG. 11B, instead of or in addition to the entry and stay regulation information shown in FIG. 11A. In addition, the activity regulation information generation unit 1001 determines an "entry-prohibited facility" included in the entry and stay regulation information in FIG. 11B based on the name of a disease/symptom (infection determination information) and the position information (information regarding the subject) of the subject which is the position information of the examination terminal 21 or the mobile terminal 31. For example, the activity regulation information generation unit 1001 holds a table showing the movable distance for each name of a disease/symptom, and determines an entry-prohibited facility based on the table, infection determination information, position information of the subject, information of the map information DB 54, information of the facility information DB 55, and the like.

The activity regulation information generation unit 1001 generates entry and exit regulation support information including the facility ID of an entry-prohibited facility shown in FIG. 11C or entry and exit regulation support information including the facility ID of an exit-prohibited facility shown in FIG. 11D, instead of or in addition to the entry and stay regulation information shown in FIGS. 11A and 11B. In addition, the activity regulation information generation unit 1001 holds a table showing the movable distance for each name of a disease/symptom, and determines an "entry-prohibited facility" included in the entry and exit regulation support information in FIG. 11C and an "exit-prohibited facility" included in the entry and exit regulation support information in FIG. 11D based on the table, infection determination information, position information of the subject, information of the map information DB 54, information of the facility information DB 55, and the like.

The activity regulation information generation unit 1001 supplies the information acquired from the infection determination unit 120 and the generated activity regulation information (entry and stay permission information) to the output unit 140.

The output unit 140 transmits the information acquired from the activity regulation information generation unit 1001 to the transmission destination terminal (the examination terminal 20 and the mobile terminal 30).

The examination terminal 21 is different from the examination terminal 20, which forms the infection spread prevention support system 1 according to the first embodiment of the present invention, in that the examination terminal 21 includes a storage unit 200 and a permission determination unit 240. Therefore, some or all of the explanation regarding units in common with the examination terminal 20 will be omitted.

The transmission and reception unit 230 receives various kinds of information from the infection spread prevention support server 11 and the like. For example, the transmission and reception unit 230 receives the entry and stay regulation information shown in FIGS. 11A and 11B or the entry and exit regulation support information shown in FIGS. 11C and 11D from the infection spread prevention support server 11. The transmission and reception unit 230 stores the received entry and stay regulation information or the entry and exit regulation support information in the storage unit 200.

The storage unit 200 stores the entry and stay regulation information or the entry and exit regulation support information received by the receiving unit 230. That is, for example, when the receiving unit 230 receives the entry and stay regulation information shown in FIG. 11A, the storage unit 200 stores entry and stay regulation information shown in FIG. 11E. In addition, for example, when the receiving unit 230 receives the entry and stay regulation information shown in FIG. 11B, the storage unit 200 stores entry and stay regulation information shown in FIG. 11F. In addition, for example, when the receiving unit 230 receives the entry and exit regulation support information shown in FIG. 11C, the storage unit 200 stores entry and exit regulation support information shown in FIG. 11G. In addition, for example, when the receiving unit 230 receives the entry and exit regulation support information shown in FIG. 11D, the storage unit 200 stores entry and exit regulation support information shown in FIG. 11H.

The permission determination unit 240 determines entry permission, stay permission, or entry and exit permission based on the activity regulation information (entry and stay regulation information and entry and exit regulation support information) stored in the storage unit 200.

For example, when it is determined that the examination terminal 21 is present in the movable range referring to the current position (position on the map) of the examination terminal 21 and the movable range shown in FIG. 11E, for example, the permission determination unit 240 determines that the stay of the place is permitted. When it is determined that the examination terminal 21 is not present in the movable range, the permission determination unit 240 determines that the stay of the place is not permitted.

In addition, for example, the transmission and reception unit 230 receives the facility ID of a facility (for example, a ticket gate), which is controlled by the activity regulation support system 71, from the facility and supplies the facility ID to the permission determination unit 240. When it is determined that the facility ID of the facility acquired from the transmission and reception unit 230 does not match the facility ID (FIG 11F) of the entry-prohibited facility stored in the storage unit 200, the permission determination unit 240 determines that the entry (for example, the passage of a ticket gate) is permitted. When it is determined that the facility ID of the facility acquired from the transmission and reception unit 230 matches the facility ID (FIG. 11F) of the entry-prohibited facility stored in the storage unit 200, the permission determination unit 240 determines that the entry (for example, the passage of a ticket gate) is not permitted.

In addition, for example, the transmission and reception unit 230 receives the facility ID of a facility (for example, a door of the hospital (communication unit facing the outdoor side)), which is controlled by the activity regulation support system 71, from the facility and supplies the facility ID to the permission determination unit 240. In the same manner as described above, when it is determined that the facility ID of the facility acquired from the transmission and reception unit 230 does not match the facility ID (FIG. 11G) of the entry-prohibited facility stored in the storage unit 200, the permission determination unit 240 determines that entry (entry into the hospital) is permitted. When it is determined that the facility ID of the facility acquired from the transmission and reception unit 230 matches the facility ID (FIG. 11G) of the entry-prohibited facility stored in the storage unit 200, the permission determination unit 240 determines that entry (entry into the hospital) is not permitted.

In addition, for example, the transmission and reception unit 230 receives the facility ID of a facility (for example, a door of the hospital (communication unit facing the indoor side)), which is controlled by the activity regulation support system 71, from the facility and supplies the facility ID to the permission determination unit 240. In the same manner as described above, when it is determined that the facility ID of the facility acquired from the transmission and reception unit 230 does not match the facility ID (FIG. 11H) of the exit-prohibited facility stored in the storage unit 200, the permission determination unit 240 determines that the exit (exit from the hospital) is permitted. When it is determined that the facility ID of the facility acquired from the transmission and reception unit 230 matches the facility ID (FIG. 11H) of the exit-prohibited facility stored in the storage unit 200, the permission determination unit 240 determines that the exit (exit from the hospital) is not permitted.

The permission determination unit 240 supplies the permission determination information, which is a result of the above-described determination, to the output unit 250.
The output unit 250 that has acquired the permission determination information from the permission determination unit 240 notifies the operator of the examination terminal 21 of the determination result and also transmits the permission determination information to the activity regulation support system 71. In addition, the activity regulation support system 71 controls the facility (for example, a ticket gate or a door of the hospital) according to the content (permitted/not permitted) of permission determination information.

In addition, the output unit 250 may include a determination result notification unit that sends a notification indicating that entry is not possible, stay is not possible, and entry and exit are not possible when permission determination information indicating "not permitted" is acquired from the permission determination unit 240 as the information of a determination result, for example. For example, the output unit 250 displays various messages as shown in FIGS. 12A to 12C. In addition, the output unit 250 may notify that entry is not possible, stay is not possible, and entry and exit are not possible by audio, or by making an LED on the main body of the examination terminal 21 emit light, or by vibrating the main body of the examination terminal 21.

The mobile terminal 31 is different from the mobile terminal 30 according to the first embodiment of the present invention in that the mobile terminal 31 includes the storage unit 300 and the permission determination unit 320. In addition, the storage unit 300, the permission determination unit 320, and the output unit 330 of the mobile terminal 31 are the same as the storage unit 200, the permission determination unit 240, and the output unit 250 of the examination terminal 21 described above.

That is, the transmission and reception unit 310 of the mobile terminal 31 receives entry and stay regulation information or entry and exit regulation support information from the infection spread prevention support server 11 and stores it in the storage unit 300. The permission determination unit 320 of the mobile terminal 31 determines entry permission, stay permission, or entry and exit permission based on the entry and stay regulation information or the entry and exit regulation support information stored in the storage unit 300, and supplies the permission determination information to the output unit 330. The output unit 330 of the mobile terminal 31 notifies the operator of the mobile terminal 31 of the determination result and also transmits the permission determination information to the activity regulation support system 71.

As described above, according to the second embodiment, it is possible to support the prevention of the spread of infectious diseases.

In addition, in the above explanation, the mode has been described in which the activity regulation information generation unit 1001 of the infection spread prevention support server 11 generates the entry and stay regulation information including the facility ID of the entry-prohibited facility as an example of the activity regulation information. However, the activity regulation information generation unit 1001 may generate entry and stay regulation information including the facility ID of the entry-allowed facility. In the mode in which the activity regulation information generation unit 1001 generates the entry and stay regulation information including the facility ID of the entry-allowed facility, when it is determined that the facility ID of the entry-allowed facility matches the facility ID of the entry-allowed facility stored in the storage unit 200, the permission determination unit 240 determines that entry (the passage of a ticket gate) is permitted. When it is determined that the facility ID of the entry-allowed facility does not match the facility ID of the entry-allowed facility stored in the storage unit 200, the permission determination unit 240 determines that entry (the passage of a ticket gate) is not permitted.

Similarly, the activity regulation information generation unit 1001 may generate entry and exit regulation information including the facility ID of the entry-allowed facility or entry and exit regulation information including the facility ID of the exit-allowed facility. In the mode in which the activity regulation information generation unit 1001 generates the entry and exit regulation information including the facility ID of the entry-allowed facility, when it is determined that the facility ID of the entry-allowed facility matches the facility ID of the entry-allowed facility stored in the storage unit 200, the permission determination unit 240 determines that entry is allowed. When it is determined that the facility ID of the entry-allowed facility does not match the facility ID of the entry-allowed facility stored in the storage unit 200, the permission determination unit 240 determines that entry is not allowed. In the mode in which the activity regulation information generation unit 1001 generates the entry and exit regulation information including the facility ID of the exit-allowed facility, when it is determined that the facility ID of the exit-allowed facility matches the facility ID of the exit-allowed facility stored in the storage unit 200, the permission determination unit 240 determines that exit is allowed. When it is determined that the facility ID of the exit-allowed facility does not match the facility ID of the exit-allowed facility stored in the storage unit 200, the permission determination unit 240 determines that exit is not allowed.

In addition, the permission determination unit 240 may determine whether or not the examination terminal 21 has moved according to a temporal change of the current position (position on the map) and calculate the moving direction and the moving speed of the examination terminal 21 based on the temporal change of the current position (position on the map). The current position of the examination terminal 21 may be acquired using a GPS, or may be acquired through communication with an access point, such as a base station or a wireless LAN.

In the mode to calculate the moving direction and the moving speed of the examination terminal 21, the permission determination unit 240 may calculate the time to go out of the movable range when the same movement as the current movement continues and supply the time to the output unit 250. In addition, in this mode, when it is determined that the examination terminal 21 is not present in the movable range, the permission determination unit 240 may calculate a direction of moving back to the movable range and supply the calculated direction to the output unit 250.

### (Third embodiment)

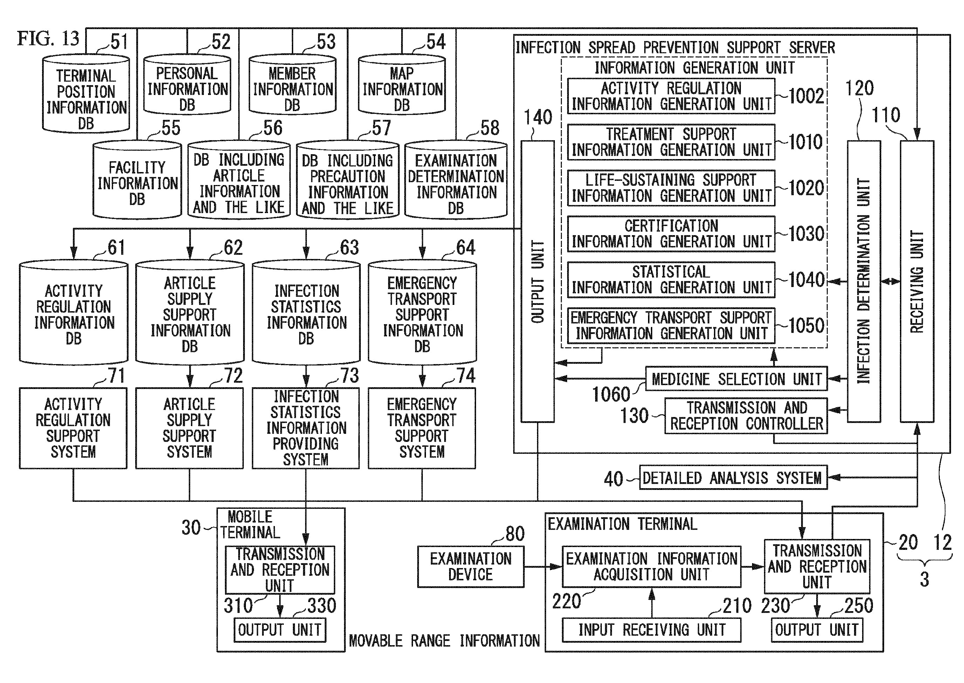

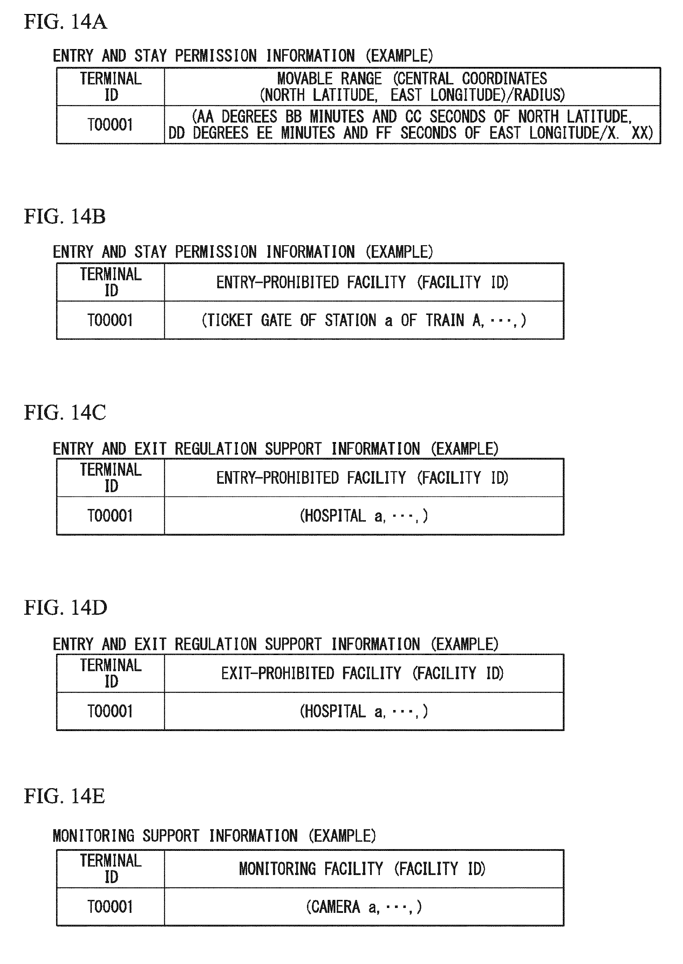

Hereinafter, a third embodiment of the present invention will be described referring to the drawings. FIG. 13 is a conceptual diagram of a system including an infection spread prevention support system according to the third embodiment of the present invention. FIGS. 14A to 14E are explanatory diagrams illustrating the infection spread prevention support system according to the third embodiment. As shown in FIG. 13, an infection spread prevention support system 3 according to the third embodiment is configured to include an infection spread prevention support server 12 and an examination terminal 20 that communicate with each other.

All of a terminal position information DB 51, a personal information DB 52, a member information DB 53, a map information DB 54, a facility information DB 55, a DB including article information and the like 56, a DB including precaution information and the like 57, an examination determination information DB 58, an activity regulation information DB 61, an article supply support information DB 62, an infection statistics information DB 63, an emergency transport support information DB 64 shown in FIG. 13 are external DBs of the infection spread prevention support system 3. In addition, all of a detailed analysis system 40, an activity regulation support system 71, an article supply support system 72, an infection statistics information providing system 73, and an emergency transport support system 74 are external DBs of the infection spread prevention support system 3. In addition, the above members other than the activity regulation information DB 61 are the same as those described in the first or second embodiment of the present invention.

A mobile terminal 30 shown in FIG. 13 is an external terminal of the infection spread prevention support system 3. The mobile terminal 30 is the same as that described in the first embodiment of the present invention. However, the output unit 330 of the mobile terminal 30 has a display function, an audio output function, a lighting function, a vibration function, and the like.

The activity regulation information DB 61 mainly stores the information generated by an activity regulation information generation unit 1002. In addition, the activity regulation information DB 61 is referred to by the activity regulation support system 71.

The infection spread prevention support server 12 is different from the infection spread prevention support servers 10 and 11 according to the first and second embodiments in that the infection spread prevention support server 12 includes the activity regulation information generation unit 1002. Therefore, some or all of the explanation regarding units in common with the infection spread prevention support servers 10 and 11 will be omitted.

The activity regulation information generation unit 1002 specifies infected patients of the infectious disease among the subjects based on infection determination information and information regarding the subject and generates activity regulation information to regulate the activities of the specified infected patients and their families. For example, as the activity regulation information, the activity regulation information generation unit 1002 generates entry and stay regulation information to regulate the entry or stay of infected patients or entry and exit regulation support information to regulate the entry and exit to and from the medical institution for the subjects.

In addition, the activity regulation information generation unit 1002 generates entry and stay regulation information including the terminal ID of a terminal to be regulated and a movable range defined by the central coordinates and the radius, for example, as shown in FIG. 14A. In addition, the "terminal ID of the terminal to be regulated" included in the entry and stay regulation information in FIG. 14A is the terminal ID (information regarding the subject) of the examination terminal 20, which is a transmission source of examination information, and/or the terminal ID (information regarding the subject) of the mobile terminal 30 owned by the subject determined to be the infected patient. In addition, the "movable range" included in the entry and stay regulation information in FIG. 14A is determined based on the name of a disease/symptom (infection determination information) and the position information (information regarding the subject) of the subject which is the position information of the examination terminal 20 or the mobile terminal 30.

In addition, for example, the activity regulation information generation unit 1002 generates entry and stay regulation information including the terminal ID of a terminal to be regulated and the facility ID of an entry-prohibited facility as shown in FIG. 14B, instead of or in addition to the entry and stay regulation information shown in FIG. 14A. In addition, the activity regulation information generation unit 1002 determines an "entry-prohibited facility" included in the entry and stay regulation information in FIG. 14B based on the name of a disease/symptom (infection determination information) and the position information (information regarding the subject) of the subject which is the position information of the examination terminal 20 or the mobile terminal 30. For example, the activity regulation information generation unit 1002 holds a table showing the movable distance for each name of a disease/symptom, and determines an entry-prohibited facility based on the table, infection determination information, position information of the subject, information of the map information DB 54, information of the facility information DB 55, and the like.

The activity regulation information generation unit 1002 generates entry and exit regulation support information including the facility ID of an entry-prohibited facility shown in FIG. 14C or entry and exit regulation support information including the facility ID of an exit-prohibited facility shown in FIG. 14D, instead of or in addition to the entry and stay regulation information shown in FIGS. 14A and 14B. In addition, the activity regulation information generation unit 1002 holds a table showing the movable distance for each name of a disease/symptom, and determines an "entry-prohibited facility" included in the entry and exit regulation support information in FIG. 14C and an "exit-prohibited facility" included in the entry and exit regulation support information in FIG. 14D based on the table, infection determination information, position information of the subject, information of the map information DB 54, information of the facility information DB 55, and the like.

In addition, the activity regulation information generation unit 1002 generates monitoring support information to support the monitoring of the subject, as the activity regulation information, as shown in FIG. 14E, instead of or in addition to the entry and stay regulation information and entry and exit regulation support information. In addition, the activity regulation information generation unit 1002 determines a "monitoring facility" included in the entry and stay regulation information in FIG. 14E based on the name of a disease/symptom (infection determination information) and the position information (information regarding the subject) of the subject which is the position information of the examination terminal 20 or the mobile terminal 30. For example, the activity regulation information generation unit 1002 holds a table showing the movable distance for each name of a disease/symptom, and determines a monitoring facility based on the table, infection determination information, position information of the subject, information of the map information DB 54, information of the facility information DB 55, and the like.

The activity regulation information generation unit 1002 supplies the information acquired from the infection determination unit 120 and the generated activity regulation information (entry and stay permission information and monitoring support information) to the output unit 140.

The output unit 140 stores (registers) the activity regulation information acquired from the activity regulation information generation unit 1002 in the activity regulation information DB 61. That is, the activity regulation information DB 61 stores the activity regulation information shown in FIGS. 14A to 14E.

The transmission and reception unit 230 of the examination terminal 20 transmits the terminal ID of the examination terminal 20 and the current position to the activity regulation support system 71. For example, when it is determined that the examination terminal 20 is present in the movable range referring to the movable range shown in FIG. 14A, which is stored in activity regulation information DB 61, the activity regulation support system 71 determines that the stay of the place is permitted. When it is determined that the examination terminal 20 is not present in the movable range, the activity regulation support system 71 determines that the stay of the place is not permitted.

In addition, for example, the transmission and reception unit 230 transmits the terminal ID of the examination terminal 20 to the facility (for example, a ticket gate) controlled by the activity regulation support system 71. The activity regulation support system 71 determines whether to permit the entry into the facility referring to the entry-prohibited facility shown in FIG. 14B stored in the activity regulation information DB 61, for example. That is, when it is determined that the facility ID of the facility, which is a communication partner of the examination terminal 20, does not match the facility ID (FIG. 14B) of the entry-prohibited facility that is stored in the activity regulation information DB 61 so as to match the terminal ID of the examination terminal 20, the activity regulation support system 71 determines that entry (passage of a ticket gate) is permitted. When it is determined that the facility ID of the facility, which is a communication partner of the examination terminal 21, matches the facility ID (FIG. 14B) of the entry-prohibited facility that is stored in the activity regulation information DB 61 so as to match the terminal ID of the examination terminal 20, the activity regulation support system 71 determines that entry (passage of a ticket gate) is not permitted.

In addition, for example, the transmission and reception unit 230 transmits the terminal ID of the examination terminal 20 to the facility (for example, a door of the hospital (communication unit facing the outdoor side)) controlled by the activity regulation support system 71. The activity regulation support system 71 determines whether to permit the entry into the facility referring to the entry-prohibited facility shown in FIG. 14C stored in the activity regulation information DB 61, for example. That is, when it is determined that the facility ID of the facility, which is a communication partner of the examination terminal 20, does not match the facility ID (FIG. 14C) of the entry-prohibited facility that is stored in the activity regulation information DB 61 so as to match the terminal ID of the examination terminal 20, the activity regulation support system 71 determines that entry (entry into the hospital) is permitted. When it is determined that the facility ID of the facility, which is a communication partner of the examination terminal 20, matches the facility ID (FIG. 14C) of the entry-prohibited facility that is stored in the activity regulation information DB 61 so as to match the terminal ID of the examination terminal 20, the activity regulation support system 71 determines that entry is not permitted.

In addition, for example, the transmission and reception unit 230 transmits the terminal ID of the examination terminal 20 to the facility (for example, a door of the hospital (communication unit facing the indoor side)) controlled by the activity regulation support system 71. The activity regulation support system 71 determines whether to permit the exit from the facility referring to the exit-prohibited facility shown in FIG. 14D stored in the activity regulation information DB 61, for example. That is, when it is determined that the facility ID of the facility, which is a communication partner of the examination terminal 20, does not match the facility ID (FIG. 14C) of the exit-prohibited facility that is stored in the activity regulation information DB 61 so as to match the terminal ID of the examination terminal 20, the activity regulation support system 71 determines that exit (exit from the hospital) is permitted. When it is determined that the facility ID of the facility, which is a communication partner of the examination terminal 20, matches the facility ID (FIG. 14C) of the exit-prohibited facility that is stored in the activity regulation information DB 61 so as to match the terminal ID of the examination terminal 20, the activity regulation support system 71 determines that exit is not permitted.

The activity regulation support system 71 controls the facility (for example, a ticket gate or a door of the hospital) according to the determination content (permitted/not permitted) and also transmits permission determination information, which is a result of the above-described determination, to the examination terminal 20. In addition, the output unit 250 of the examination terminal 20 may include a determination result notification unit that sends a notification indicating that entry is not possible, stay is not possible, and entry and exit are not possible by audio, or by making an LED on the main body of the examination terminal 21 emit light, or by vibrating the main body of the examination terminal 21 when permission determination information indicating "not permitted" is acquired from the permission determination unit 240.

In addition, the activity regulation support system 71 drives a monitoring camera, which monitors subjects or their families, referring to the monitoring support information shown in FIG. 14E stored in the activity regulation information DB 61, for example. For example, the activity regulation support system 71 matches the direction of the monitoring camera to the direction of the current position of an infected patient.

As described above, according to the third embodiment, it is possible to support the prevention of the spread of infectious diseases.

In addition, in the above explanation, the mode has been described in which the activity regulation information generation unit 1002 of the infection spread prevention support server 12 generates the entry and stay regulation information including the facility ID of the entry-prohibited facility as an example of the activity regulation information. However, the activity regulation information generation unit 1002 may generate entry and stay regulation information including the facility ID of the entry-allowed facility. In the mode in which the activity regulation information generation unit 1002 generates the entry and stay regulation information including the facility ID of the entry-allowed facility, when it is determined that the facility ID of the facility, which is a communication partner of the examination terminal 20, matches the facility ID of the entry-allowed facility stored in the activity regulation information DB 61 so as to match the facility ID of the examination terminal 20, the activity regulation support system 71 determines that entry is permitted. When it is determined that the facility ID of the facility, which is a communication partner of the examination terminal 20, does not match the facility ID of the entry-allowed facility stored in the activity regulation information DB 61 so as to match the facility ID of the examination terminal 20, the activity regulation support system 71 determines that entry is not permitted.

Similarly, the activity regulation information generation unit 1002 may generate entry and exit regulation information including the facility ID of the entry-allowed facility or entry and exit regulation information including the facility ID of the exit-allowed facility. In the mode in which the activity regulation information generation unit 1002 generates the entry and exit regulation information including the facility ID of the entry-allowed facility, when it is determined that the facility ID of the facility, which is a communication partner of the examination terminal 20, matches the facility ID of the entry-allowed facility stored in the activity regulation information DB 61 so as to match the facility ID of the examination terminal 20, the activity regulation support system 71 determines that entry is permitted. When it is determined that the facility ID of the facility, which is a communication partner of the examination terminal 20, does not match the facility ID of the entry-allowed facility stored in the activity regulation information DB 61 so as to match the facility ID of the examination terminal 20, the activity regulation support system 71 determines that entry is not permitted. In the mode in which the activity regulation information generation unit 1002 generates the entry and exit regulation information including the facility ID of the exit-allowed facility, when it is determined that the facility ID of the facility, which is a communication partner of the examination terminal 20, matches the facility ID of the exit-allowed facility stored in the activity regulation information DB 61 so as to match the facility ID of the examination terminal 20, the activity regulation support system 71 determines that exit is permitted. When it is determined that the facility ID of the facility, which is a communication partner of the examination terminal 20, does not match the facility ID of the exit-allowed facility stored in the activity regulation information DB 61 so as to match the facility ID of the examination terminal 20, the activity regulation support system 71 determines that exit is not permitted.

In addition, the activity regulation support system 71 may determine whether or not the examination terminal 20 has moved according to a temporal change of the current position (position on the map) and calculate the moving direction and the moving speed of the examination terminal 20 based on the temporal change of the current position (position on the map).

In the mode to calculate the moving direction and the moving speed of the examination terminal 21, the activity regulation support system 71 may calculate the time to go out of the movable range when the same movement as the current movement continues and supply the time to the examination terminal 20. In addition, in this mode, when it is determined that the examination terminal 20 is not present in the movable range, the activity regulation support system 71 may calculate a direction of moving back to the movable range and transmit the calculated direction to the examination terminal 20.

In addition, in the above explanation, the mode has been described in which the activity regulation support system 71 matches the direction of the monitoring camera to the direction of the current position of the infected patient. However, the mode to control the direction of the monitoring camera is not limited to this. For example, by storing (accumulating) the past activity information of each subject in the personal information DB 52, the activity regulation support system 71 may predict activity patterns based on the past activity patterns of the infected patient and control the monitoring camera according to the prediction result. In addition, for example, by counting the activity history of the subject for the same area and the same attribute in the personal information DB 52, the activity regulation support system 71 may predict the activity patterns of the infected patient based on the activity patterns of infected patients with similar characteristics and control the monitoring camera according to the prediction result.

That is, in the above explanation, the mode has been described in which the activity regulation information generation unit 1002 generates the monitoring support information including the facility ID of the monitoring facility as an example. However, the activity regulation information generation unit 1002 may generate monitoring support information including the control information of the monitoring facility (for example, direction information of the monitoring camera) as described above.

In addition, the activity regulation support system 71 may have a biological recognition function, such as a face recognition function. When the activity regulation support system 71 has a biological recognition function, the activity regulation support system 71 specifies a subject in a captured image referring to a captured image, which is captured by a monitoring camera, and biological recognition information of the personal information DB 52 (feature amount data of the face). That is, the activity regulation support system 71 monitors each infected patient using a monitoring camera.

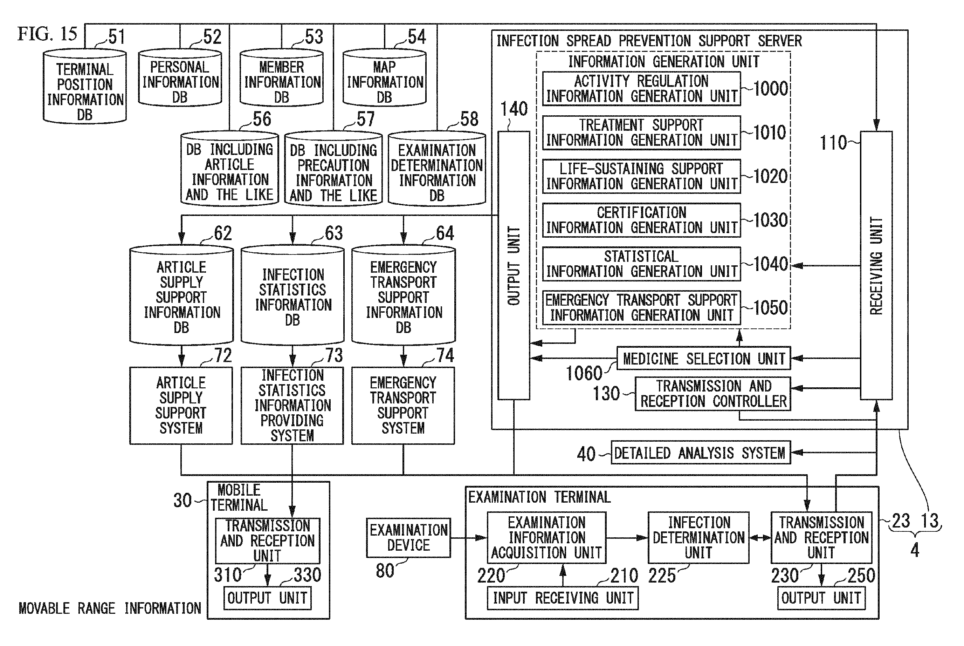

In addition, in the explanation of the first embodiment, as shown in FIG. 1, the mode has been described in which the examination terminal 20 transmits the examination information to the infection spread prevention support server 10 and the infection spread prevention support server 10 determines the presence or degree of infection of an infectious disease. However, as shown in FIG. 15, an examination terminal 23 may determine the presence or degree of infection of an infectious disease and transmit the infection determination information to an infection spread prevention support server 13. An infection spread prevention support system 4 shown in FIG. 15 is configured to include the infection spread prevention support server 13 and the examination terminal 23 that communicate with each other. The examination terminal 23 includes a mounting unit (not shown) for mounting an examination device 80, an input receiving unit 210, an examination information acquisition unit 220, an infection determination unit 225, a transmission and reception unit 230, and an output unit 250. The infection spread prevention support server 13 includes a receiving unit 110, a transmission and reception controller 130, an activity regulation information generation unit 1000, a treatment support information generation unit 1010, a life-sustaining support information generation unit 1020, a certification information generation unit 1030, a statistical information generation unit 1040, an emergency transport support information generation unit 1050, a medicine selection unit 1060, and an output unit 140. In addition, the infection determination unit 225 can perform the same process as in the infection determination unit 120 in a first embodiment by acquiring the examination determination information stored in the examination determination information DB 58, for example, through the transmission and reception unit 230 and comparing the acquired examination determination information with the examination information of the examination information acquisition unit 220.

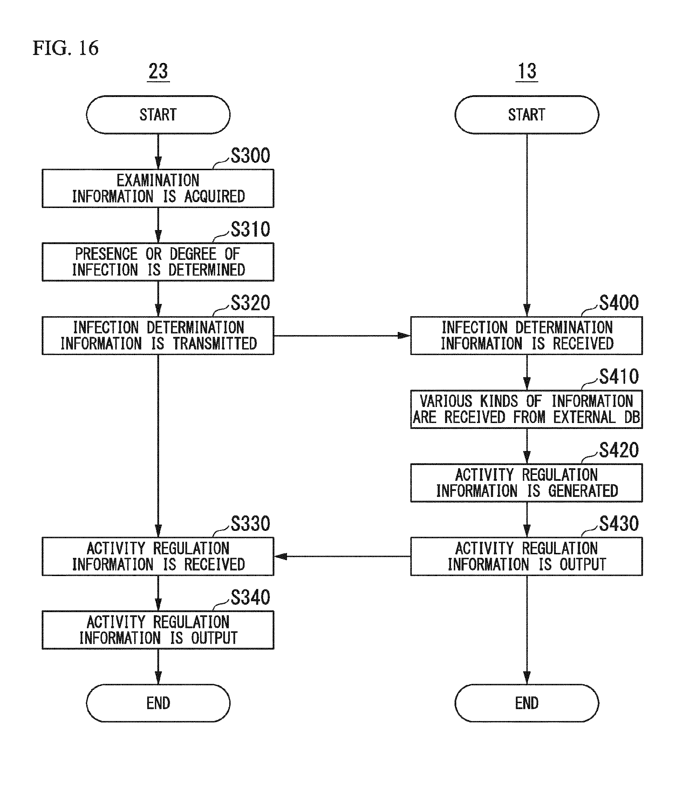

The flow chart shown in FIG. 16 is an example in which, in the configuration shown in FIG. 15, the infection spread prevention support server 13 generates activity regulation information and transmits the generated activity regulation information to the examination terminal 23. In addition, in FIG. 16, the left side shows the process of the examination terminal 23, and the right side shows the process of the infection spread prevention support server 13.

The examination information acquisition unit 220 of the examination terminal 23 acquires examination information to examine the infection of infectious diseases (step S300). The infection determination unit 225 of the examination terminal 23 determines the presence or degree of infection of an infectious disease using the examination information (step S310). The transmission and reception unit 230 of the examination terminal 23 transmits the infection determination information to the infection spread prevention support server 13 (step S320). In addition, when the input information is acquired from the input receiving unit 210, the transmission and reception unit 230 of the examination terminal 23 transmits the input information and the infection determination information to the infection spread prevention support server 13.

The receiving unit 110 of the infection spread prevention support server 13 receives the information transmitted from the examination terminal 20 (step S400). The receiving unit 110 of the infection spread prevention support server 13 receives information, which is required to generate activity regulation information, from the external DB (step S410). The activity regulation information generation unit 1000 of the infection spread prevention support server 13 generates activity regulation information to regulate the activities of an infected patient based on the infection determination information and the information regarding the subject (step S420). The output unit 140 of the infection spread prevention support server 13 transmits the activity regulation information to the examination terminal 23 (step S430).

The transmission and reception unit 230 of the examination terminal 23 receives the activity regulation information transmitted from the infection spread prevention support server 10 (step S330). The output unit 250 of the examination terminal 23 displays the activity regulation information (step S340). Then, this flow chart is ended.

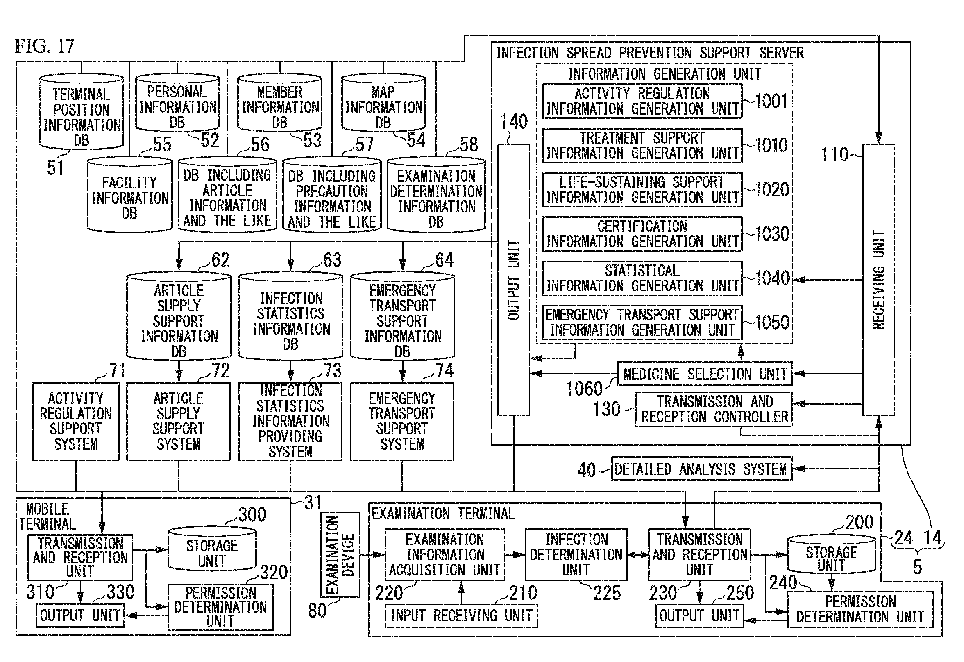

Similarly, in the explanation of the second embodiment, as shown in FIG. 10, the mode has been described in which the examination terminal 21 transmits the examination information to the infection spread prevention support server 11 and the infection spread prevention support server 11 determines the presence or degree of infection of an infectious disease. However, as shown in FIG. 17, an examination terminal 24 may determine the presence or degree of infection of an infectious disease and transmit the infection determination information to an infection spread prevention support server 14. An infection spread prevention support system 5 shown in FIG. 17 is configured to include the infection spread prevention support server 14 and the examination terminal 24 that communicate with each other. The examination terminal 24 includes a mounting unit (not shown) for mounting an examination device 80, a storage unit 200, an input receiving unit 210, an examination information acquisition unit 220, an infection determination unit 225, a transmission and reception unit 230, a permission determination unit 240, and an output unit 250. The infection spread prevention support server 14 includes a receiving unit 110, a transmission and reception controller 130, an activity regulation information generation unit 1001, a treatment support information generation unit 1010, a life-sustaining support information generation unit 1020, a certification information generation unit 1030, a statistical information generation unit 1040, an emergency transport support information generation unit 1050, a medicine selection unit 1060, and an output unit 140.

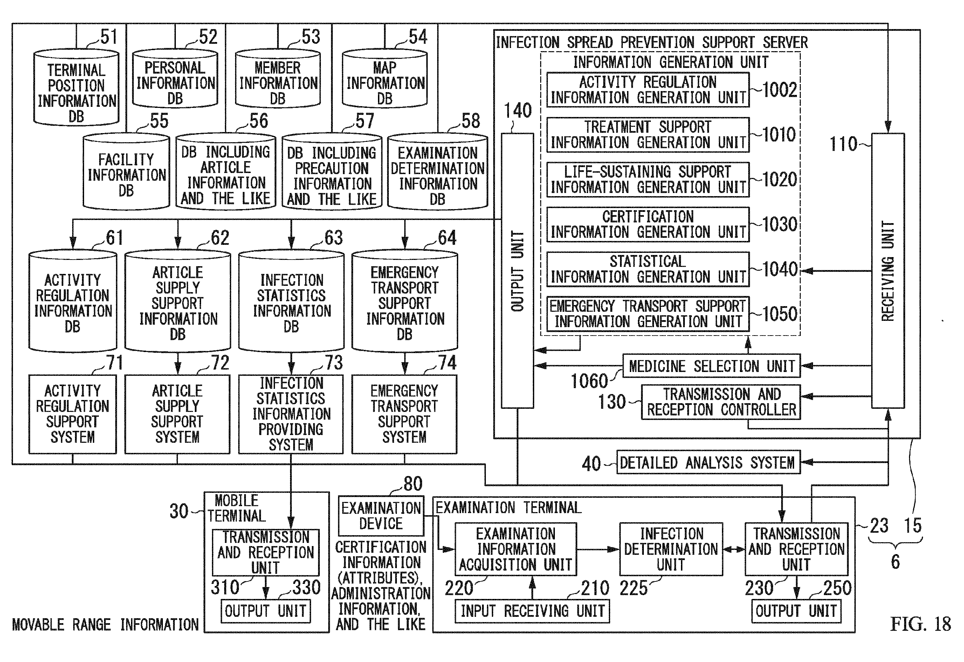

Similarly, in the explanation of the third embodiment, as shown in FIG. 13, the mode has been described in which the examination terminal 20 transmits the examination information to the infection spread prevention support server 12 and the infection spread prevention support server 12 determines the presence or degree of infection of an infectious disease. However, as shown in FIG. 18, an examination terminal 23 may determine the presence or degree of infection of an infectious disease and transmit the infection determination information to an infection spread prevention support server 15. An infection spread prevention support system 6 shown in FIG. 18 is configured to include the infection spread prevention support server 15 and the examination terminal 23 that communicate with each other. The examination terminal 23 includes a mounting unit (not shown) for mounting an examination device 80, an input receiving unit 210, an examination information acquisition unit 220, an infection determination unit 225, a transmission and reception unit 230, and an output unit 250. The infection spread prevention support server 15 includes a receiving unit 110, a transmission and reception controller 130, an activity regulation information generation unit 1002, a treatment support information generation unit 1010, a life-sustaining support information generation unit 1020, a certification information generation unit 1030, a statistical information generation unit 1040, an emergency transport support information generation unit 1050, a medicine selection unit 1060, and an output unit 140.

In addition, in the present embodiment, the above-described examination terminal may be configured to have a function of the above-described mobile terminal that is portable or mobile. In addition, in the present embodiment, the above-described mobile terminal may be configured to have a function (for example, an examination information acquisition unit or an infection determination unit) of the above-described examination terminal. In addition, the information (for example, activity regulation information, treatment support information, and life-sustaining support information) generated by the information generation unit in the present embodiment may be stored in a storage unit (for example, a memory or a database) in the above-described server.

In addition, the present embodiment may also be used in medical institutions, such as hospitals. In the case of a medical institution, the activity range of an infected patient may be regulated by activity regulation information in the outpatients' ward and the inpatients' ward (or the isolation ward and other wards), or treatment support for an inpatient of an infectious disease may be offered by treatment support information, or the life-sustaining support information may be output to an electronic chart system, a mobile terminal, and the like.

In addition, each process of the infection spread prevention support servers 10, 11, and 12, each process of the examination terminals 20 and 21, and the various processes related to the mobile terminals 30 and 31 may be performed by recording a program for executing each process of the infection spread prevention support servers 10, 11, and 12, each process of the examination terminals 20 and 21, and each process of the mobile terminals 30 and 31 in a computer-readable recording medium, reading the program recorded in the recording medium into a computer system, and executing the read program. In addition, the "computer system" referred to herein may include an OS or hardware, such as peripheral apparatuses. In addition, the "computer system" may also include a homepage presenting environment (or display environment) if a WWW system is used. In addition, the "computer-readable recording medium" refers to writable nonvolatile memories such as a flexible disk, a magneto-optical disc, a ROM, and a flash memory, portable media such as a CD-ROM, and a storage unit such as a hard disk built in a computer system.

In addition, of the "computer-readable recording medium" also includes a medium that stores a program for a predetermined period of time, such as volatile memory (for example, a DRAM (Dynamic Random Access Memory)) in a computer system serving as a server or a client when the program is transmitted through a network, such as Internet, or a communication line, such as a telephone line. In addition, the program may be transmitted from a computer system, which has a storage unit or the like that stores the program, to other computer systems through a transmission medium or a transmission wave in the transmission medium. Here, the "transmission medium" that transmits a program refers to a medium having a function of transmitting information, such as a network (communication network) including the Internet or a communication line including a telephone line. In addition, the above-described program may be provided to realize a part of the function described above. In addition, the program may be a so-called differential file (differential program) that can realize the above function in combination with a program already recorded in a computer system.

While the embodiments of the invention have been described in detail referring to the drawings, the specific configuration is not limited to the above-described embodiments but design and the like within the scope without departing from the subject matter of the invention are also included.

### Reference Signs List

1, 2, 3, 4, 5, 6: infection spread prevention support system
10, 11, 12, 13, 14, 15: infection spread prevention support server
20, 21, 23, 24: examination terminal
30, 31: mobile terminal
40: detailed analysis system
51: terminal position information DB
52: personal information DB
53: member information DB
54: map information DB
55: facility information DB
56: DB including article information and the like
57: DB including precaution information and the like
58: examination determination information DB
61: activity regulation information DB
62: article supply support information DB
63: infection statistics information DB
64: emergency transport support information DB
71: activity regulation support system
72: article supply support system
73: infection statistics information providing system
74: emergency transport support system
80: examination device
110: receiving unit
120: infection determination unit
130: transmission and reception controller
140: output unit
200: storage unit
210: input receiving unit
220: examination information acquisition unit
225: infection determination unit
230: transmission and reception unit
240: permission determination unit
250: output unit
300: storage unit
310: transmission and reception unit
320: permission determination unit
330: output unit
1000, 1001, 1002: activity regulation information generation unit
1010: treatment support information generation unit
1020: life-sustaining support information generation unit
1030: certification information generation unit
1040: statistical information generation unit
1050: emergency transport support information generation unit
1060: medicine selection unit

## Claims

1. An infection spread prevention support system comprising:
an examination terminal; and
a server,
wherein the examination terminal includes:
an examination information acquisition unit that acquires examination information to examine infection of an infectious disease in subjects; and
a transmission unit that transmits the examination information to the server, and
the server includes:
a receiving unit that receives the examination information and information regarding the subjects;
an infection determination unit configured to determine presence or degree of infection of the infectious disease by comparing the examination information with examination determination information stored in a storage unit;
an activity regulation information generation unit configured to specify an infected patient of the infectious disease among the subjects based on infection determination information, which is a determination result of the infection determination unit, and the information regarding the subjects and to generate activity regulation information to regulate an activity of the infected patient; and
an output unit that outputs the activity regulation information of the infected patient.

2. An infection spread prevention support system comprising:
an examination terminal; and
a server,
wherein the examination terminal includes:
an examination information acquisition unit that acquires examination information to examine infection of an infectious disease in subjects;
an infection determination unit configured to determine presence or degree of infection of the infectious disease by comparing the examination information with examination determination information stored in a storage unit; and
a transmission unit that transmits infection determination information, which is a determination result of the infection determination unit, to the server, and
the server includes:
a receiving unit that receives the infection determination information and information regarding the subjects;
an activity regulation information generation unit configured to specify an infected patient of the infectious disease among the subjects based on the infection determination information and the information regarding the subjects and to generate activity regulation information to regulate an activity of the infected patient; and
an output unit that outputs the activity regulation information of the infected patient.

3. An infection spread prevention support system comprising:
an examination terminal; and
a server,
wherein the examination terminal includes:
an examination information acquisition unit that acquires examination information to examine infection of an infectious disease in subjects; and
a transmission unit that transmits the examination information to the server, and
the server includes:
a receiving unit that receives the examination information and information regarding the subjects;
an infection determination unit configured to determine presence or degree of infection of the infectious disease by comparing the examination information with examination determination information stored in a storage unit;
a treatment support information generation unit configured to specify an infected patient of the infectious disease among the subjects based on infection determination information, which is a determination result of the infection determination unit, and the information regarding the subjects and to generate treatment support information to support treatment of the infected patient; and
an output unit that outputs the treatment support information of the infected patient.

4. An infection spread prevention support system comprising:
an examination terminal; and
a server,
wherein the examination terminal includes:
an examination information acquisition unit that acquires examination information to examine infection of an infectious disease in subjects;
an infection determination unit configured to determine presence or degree of infection of the infectious disease by comparing the examination information with examination determination information stored in a storage unit; and
a transmission unit that transmits infection determination information, which is a determination result of the infection determination unit, to the server, and
the server includes:
a receiving unit that receives the infection determination information and information regarding the subjects;
a treatment support information generation unit configured to specify an infected patient of the infectious disease among the subjects based on the infection determination information and the information regarding the subjects and to generate treatment support information to support treatment of the infected patient; and
an output unit that outputs the treatment support information of the infected patient.

5. An infection spread prevention support system comprising:
an examination terminal; and
a server,
wherein the examination terminal includes:
an examination information acquisition unit that acquires examination information to examine infection of an infectious disease in subjects; and
a transmission unit that transmits the examination information to the server, and
the server includes:
a receiving unit that receives the examination information and information regarding the subjects;
an infection determination unit configured to determine presence or degree of infection of the infectious disease by comparing the examination information with examination determination information stored in a storage unit;
a life-sustaining support information generation unit configured to specify an infected patient of the infectious disease among the subjects based on infection determination information, which is a determination result of the infection determination unit, and the information regarding the subjects and to generate life-sustaining support information to support maintenance of life of the infected patient; and
an output unit that outputs the life-sustaining support information of the infected patient.

6. An infection spread prevention support system comprising:
an examination terminal; and
a server,
wherein the examination terminal includes:
an examination information acquisition unit that acquires examination information to examine infection of an infectious disease in subjects;
an infection determination unit configured to determine presence or degree of infection of the infectious disease by comparing the examination information with examination determination information stored in a storage unit; and
a transmission unit that transmits infection determination information, which is a determination result of the infection determination unit, to the server, and
the server includes:
a receiving unit that receives the infection determination information and information regarding the subjects;
a life-sustaining support information generation unit configured to specify an infected patient of the infectious disease among the subjects based on the infection determination information and the information regarding the subjects and to generate life-sustaining support information to support maintenance of life of the infected patient; and
an output unit that outputs the life-sustaining support information of the infected patient.

7. An infection spread prevention support server comprising:
a receiving unit that receives examination information to examine infection of an infectious disease in subjects and information regarding the subjects;
an infection determination unit configured to determine presence or degree of infection of the infectious disease by comparing the examination information with examination determination information stored in a storage unit;
an activity regulation information generation unit configured to specify an infected patient of the infectious disease among the subjects based on infection determination information, which is a determination result of the infection determination unit, and the information regarding the subjects and to generate activity regulation information to regulate an activity of the infected patient; and
an output unit that outputs the activity regulation information of the infected patient.

8. An infection spread prevention support server comprising:
a receiving unit that receives infection determination information, which is a determination result when presence or degree of infection of an infectious disease is determined by comparing examination information to examine infection of the infectious disease in subjects with examination determination information stored in a storage unit, and information regarding the subjects;
an activity regulation information generation unit configured to specify an infected patient of the infectious disease among the subjects based on the infection determination information and the information regarding the subjects and to generate activity regulation information to regulate an activity of the infected patient; and
an output unit that outputs the activity regulation information of the infected patient.

9. The infection spread prevention support server according to claim 7 or 8,
wherein the activity regulation information generation unit is configured to generate movement restriction information, which indicates a movable range of the infected patient, as the activity regulation information.

10. The infection spread prevention support server according to any one of claims 7 to 9,
wherein the activity regulation information generation unit is configured to generate entry and stay regulation information, which is for regulating entry or stay of the infected patient, as the activity regulation information.

11. The infection spread prevention support server according to any one of claims 7 to 10,
wherein the activity regulation information generation unit is configured to generate entry and exit regulation support information, which is for regulating entry and exit to and from a medical institution for the subjects, as the activity regulation information.

12. The infection spread prevention support server according to any one of claims 7 to 11,
wherein the activity regulation information generation unit is configured to generate monitoring support information, which is for supporting monitoring of the infected patient, as the activity regulation information.

13. The infection spread prevention support server according to any one of claims 7 to 12,
wherein the activity regulation information generation unit is configured to generate precaution statement and action guideline information, which indicates a precaution statement or an action guideline offered to the subjects, as the activity regulation information.

14. The infection spread prevention support server according to any one of claims 7 to 13,
wherein the activity regulation information generation unit is configured to generate activity plan information, which indicates an activity plan of authorities who prevent spread of the infectious disease, as the activity regulation information.

15. The infection spread prevention support server according to any one of claims 7 to 14,
wherein the activity regulation information generation unit is configured to generate the activity regulation information according to attributes of the subjects based on the infection determination information and information regarding the subjects including attribute information of the subjects.

16. The infection spread prevention support server according to claim 7 or 15,
wherein the activity regulation information generation unit is configured to generate article supply support information, which is for supporting supply of an article used to prevent spread of infection of the infectious disease, based on the infection determination information and the information regarding the subjects.

17. The infection spread prevention support server according to any one of claims 7 to 16,
wherein the activity regulation information generation unit is configured to generate activity regulation lifting information, which is for lifting activity regulation of the infected patient, based on the infection determination information and the information regarding the subjects.

18. The infection spread prevention support server according to any one of claims 7 to 17,
wherein the output unit outputs the activity regulation information based on member information, which is member information regarding a member to whom the activity regulation information is provided and which includes a designated area and a designated output destination that are designated by each member.

19. An infection spread prevention support server comprising:
a receiving unit that receives examination information to examine infection of an infectious disease in subjects and information regarding the subjects;
an infection determination unit configured to determine presence or degree of infection of the infectious disease by comparing the examination information with examination determination information stored in a storage unit;
a treatment support information generation unit configured to specify an infected patient of the infectious disease among the subjects based on infection determination information, which is a determination result of the infection determination unit, and the information regarding the subjects and to generate treatment support information to support treatment of the infected patient; and
an output unit that outputs the treatment support information of the infected patient.

20. An infection spread prevention support server comprising:
a receiving unit that receives infection determination information, which is a determination result when presence or degree of infection of an infectious disease is determined by comparing examination information to examine infection of the infectious disease in subjects with examination determination information stored in a storage unit, and information regarding the subjects;
a treatment support information generation unit configured to specify an infected patient of the infectious disease among the subjects based on the infection determination information and the information regarding the subjects and to generate treatment support information to support treatment of the infected patient; and
an output unit that outputs the treatment support information of the infected patient.

21. The infection spread prevention support server according to claim 19 or 20,
wherein the treatment support information generation unit is configured to generate article supply support information, which is for supporting supply of an article used in treatment of the infected patient, as the treatment support information.

22. The infection spread prevention support server according to any one of claims 19 to 21,
wherein the treatment support information generation unit is configured to generate precaution statement and action guideline information, which indicates a precaution statement or an action guideline offered to the infected patient, as the treatment support information.

23. The infection spread prevention support server according to any one of claims 19 to 22,
wherein the treatment support information generation unit is configured to generate activity plan information, which indicates an activity plan of authorities regarding prevention of spread of the infectious disease, as the treatment support information.

24. The infection spread prevention support server according to any one of claims 19 to 23,
wherein the treatment support information generation unit is configured to generate the treatment support information according to attributes of the subjects based on the infection determination information and information regarding the subjects including attribute information of the subjects.

25. The infection spread prevention support server according to any one of claims 19 to 24,
wherein the output unit outputs the treatment support information based on member information, which is member information regarding a member to whom the treatment support information is provided and which includes a designated area and a designated output destination that are designated by each member.

26. An infection spread prevention support server comprising:
a receiving unit that receives examination information to examine infection of an infectious disease in subjects and information regarding the subjects;
an infection determination unit configured to determine presence or degree of infection of the infectious disease by comparing the examination information with examination determination information stored in a storage unit;
a life-sustaining support information generation unit configured to specify an infected patient of the infectious disease among the subjects based on infection determination information, which is a determination result of the infection determination unit, and the information regarding the subjects and to generate life-sustaining support information to support maintenance of life of the infected patient; and
an output unit that outputs the life-sustaining support information of the infected patient.

27. An infection spread prevention support server comprising:
a receiving unit that receives infection determination information, which is a determination result when presence or degree of infection of an infectious disease is determined by comparing examination information to examine infection of the infectious disease in subjects with examination determination information stored in a storage unit, and information regarding the subjects;
a life-sustaining support information generation unit configured to specify an infected patient of the infectious disease among the subjects based on the infection determination information and the information regarding the subjects and to generate life-sustaining support information to support maintenance of life of the infected patient; and
an output unit that outputs the life-sustaining support information of the infected patient.

28. The infection spread prevention support server according to claim 26 or 27,
wherein the life-sustaining support information generation unit is configured to generate article supply support information, which is for supporting supply of an article required to maintain life of the infected patient, as the life-sustaining support information.

29. The infection spread prevention support server according to any one of claims 26 to 28,
wherein the life-sustaining support information generation unit is configured to generate precaution statement and action guideline information, which indicates a precaution statement or an action guideline offered to the infected patient, as the life-sustaining support information.

30. The infection spread prevention support server according to any one of claims 26 to 29,
wherein the life-sustaining support information generation unit is configured to generate activity plan information, which indicates an activity plan of authorities regarding prevention of spread of the infectious disease, as the life-sustaining support information.

31. The infection spread prevention support server according to any one of claims 26 to 30,
wherein the life-sustaining support information generation unit is configured to generate the life-sustaining support information according to attributes of the subjects based on the infection determination information and information regarding the subjects including attribute information of the subjects.

32. The infection spread prevention support server according to any one of claims 26 to 31,
wherein the output unit outputs the life-sustaining support information based on member information, which is member information regarding a member to whom the life-sustaining support information is provided and which includes a designated area and a designated output destination that are designated by each member.

33. The infection spread prevention support server according to any one of claims 7 to 18, further comprising:
a treatment support information generation unit configured to generate treatment support information, which is for supporting treatment of the infectious disease, based on the infection determination information and the information regarding the subjects,
wherein the output unit outputs the treatment support information.

34. The infection spread prevention support server according to any one of claims 7 to 18, further comprising:
a life-sustaining support information generation unit configured to generate life-sustaining support information, which is for supporting maintenance of life of the infected patient, based on the infection determination information and the information regarding the subjects,
wherein the output unit outputs the life-sustaining support information.

35. The infection spread prevention support server according to any one of claims 19 to 25, further comprising:
an activity regulation information generation unit configured to generate activity regulation information, which is for regulating an activity of the infected patient, based on the infection determination information and the information regarding the subjects,
wherein the output unit outputs the activity regulation information.

36. The infection spread prevention support server according to any one of claims 19 to 25, further comprising:
a life-sustaining support information generation unit configured to generate life-sustaining support information, which is for supporting maintenance of life of the infected patient, based on the infection determination information and the information regarding the subjects,
wherein the output unit outputs the life-sustaining support information.

37. The infection spread prevention support server according to any one of claims 26 to 32, further comprising:
an activity regulation information generation unit configured to generate activity regulation information, which is for regulating an activity of the infected patient, based on the infection determination information and the information regarding the subjects,
wherein the output unit outputs the activity regulation information.

38. The infection spread prevention support server according to any one of claims 26 to 32, further comprising:
a treatment support information generation unit configured to generate treatment support information, which is for supporting treatment of the infectious disease, based on the infection determination information and the information regarding the subjects,
wherein the output unit outputs the treatment support information.

39. The infection spread prevention support server according to any one of claims 7 to 38, further comprising:
a certification information generation unit configured to generate certification information, which includes the infection determination information and determination date and time, based on the infection determination information and the information regarding the subjects.

40. The infection spread prevention support server according to any one of claims 7 to 39, further comprising:
an infection statistics information generation unit configured to generate infection statistics information for each area based on the infection determination information and the information regarding the subjects.

41. The infection spread prevention support server according to claim 40,
wherein the infection statistics information generation unit is configured to generate infection statistics information for each area and each attribute of the subjects based on the infection determination information and information regarding the subjects including attribute information of the subjects.

42. The infection spread prevention support server according to any one of claims 7 to 41, further comprising:
an emergency transport support information generation unit configured to generate emergency transport support information, which is for supporting transport of the infected patient, based on the infection determination information and the information regarding the subjects.

43. The infection spread prevention support server according to any one of claims 7 to 42, further comprising:
a medicine selection unit configured to select a medicine, which is used in treatment of the infected patient, based on the infection determination information and the information regarding the subjects.

44. The infection spread prevention support server according to any one of claims 7 to 43,
wherein the examination information is examination information including personal identification information that identifies the subject, and the infection determination unit is configured to perform an identification process of the subject based on the personal identification information of the subject stored in advance and the personal identification information acquired from the examination information and to determine the presence or degree of infection of the infectious disease using the examination information of the subject.

45. The infection spread prevention support server according to any one of claims 7 to 44,
wherein the infection determination unit is configured to determine the presence or degree of infection of the infectious disease referring to administration information regarding medicine that the subject takes.

46. The infection spread prevention support server according to any one of claims 7 to 45, further comprising:
a transmission and reception controller configured to perform at least one of control to selectively transmit information to the examination terminal, control to selectively output information to the output unit, and control to selectively receive information transmitted from the examination terminal, based on the infection determination information.

47. An examination terminal comprising:
an examination information acquisition unit that acquires examination information used to determine presence or degree of infection of an infectious disease of a subject;
an infection determination unit configured to determine the presence or degree of infection of the infectious disease by comparing the examination information with examination determination information stored in a storage unit; and
a transmission unit that transmits infection determination information, which is a determination result of the infection determination unit, to a server.

48. The examination terminal according to claim 47, further comprising:
a mounting unit of an examination device that examines infection of the infectious disease,
wherein the examination information acquisition unit acquires the examination information from the examination device.

49. The examination terminal according to claim 47 or 48, further comprising:
a receiving unit that receives from the server at least one of activity regulation information to regulate an activity of an infected patient of the infectious disease among the subjects, treatment support information to support treatment of the infectious disease, and life-sustaining support information to support maintenance of life of the infected patient; and
an output unit that outputs the information received by the receiving unit.

50. The examination terminal according to claim 47 or 48, further comprising:
a receiving unit that receives entry and stay regulation information to regulate entry or stay of the infected patient or entry and exit regulation support information to regulate entry and exit to and from a medical institution for the subjects, as activity regulation information to regulate an activity of the infected patient of the infectious disease among the subjects, from the server;
a storage unit that stores the activity regulation information;
a permission determination unit configured to determine whether or not entry is permitted, whether or not stay is permitted, or whether or not entry and exit are permitted based on the activity regulation information; and
a determination result notification unit that sends a notification of a determination result of the permission determination unit.

51. The examination terminal according to claim 47 or 48, further comprising:
a receiving unit that receives a determination result regarding whether or not entry is permitted, whether or not stay is permitted, or whether or not entry and exit are permitted, which is based on activity regulation information generated by the server; and
a determination result notification unit that sends a notification of the determination result.

52. A mobile terminal comprising:
a receiving unit that receives, from a server configured to generate activity regulation information to regulate an activity of an infected patient of an infectious disease among subjects, entry and stay regulation information to regulate entry or stay of the infected patient or entry and exit regulation support information to regulate entry and exit to and from a medical institution for the subjects, as the activity regulation information, based on infection determination information indicating presence or degree of infection of the infectious disease in the subjects;
a storage unit that stores the activity regulation information;
a permission determination unit configured to determine whether or not entry is permitted, whether or not stay is permitted, or whether or not entry and exit are permitted based on the activity regulation information; and
a determination result notification unit that sends a notification of a determination result of the permission determination unit.

53. A mobile terminal comprising:
a receiving unit that receives a determination result regarding whether or not entry is permitted, whether or not stay is permitted, or whether or not entry and exit are permitted, which is based on activity regulation information for regulating an activity of an infected patient that is generated by a server based on infection determination information indicating presence or degree of infection of an infectious disease in a subject; and
a determination result notification unit that sends a notification of the determination result.

54. The mobile terminal according to claim 52 or 53, further comprising:
an examination information acquisition unit that acquires the examination information; and
a transmission unit that transmits the examination information acquired by the examination information acquisition unit to the server.

55. The mobile terminal according to claim 52 or 53, further comprising:
an examination information acquisition unit that acquires the examination information;
an infection determination unit configured to determine the presence or degree of infection of the infectious disease by comparing the examination information acquired by the examination information acquisition unit with examination determination information stored in a storage unit; and
a transmission unit that transmits the infection determination information, which is a determination result of the infection determination unit, to the server.

56. A program causing a computer of an infection spread prevention support server to execute:
a receiving step of receiving examination information to examine infection of an infectious disease in subjects and information regarding the subjects;
an infection determination step of determining presence or degree of infection of the infectious disease by comparing the examination information with examination determination information stored in a storage unit;
a step of specifying an infected patient of the infectious disease among the subjects based on infection determination information, which is a determination result in the infection determination step, and the information regarding the subjects;
an activity regulation information generation step of generating activity regulation information to regulate an activity of the infected patient; and
an output step of outputting the activity regulation information.

57. A program causing a computer of an examination terminal having a mounting unit of an examination device that examines infection of an infectious disease in a subject to execute:
an examination information acquisition step of acquiring examination information used to determine presence or degree of infection of the infectious disease;
an infection determination step of determining the presence or degree of infection of the infectious disease by comparing the examination information with examination determination information stored in a storage unit;
a transmission step of transmitting infection determination information, which is a determination result in the infection determination step, to a server;
a receiving step of receiving from the server at least one of activity regulation information to regulate an activity of an infected patient of the infectious disease, treatment support information to support treatment of the infected patient, and life-sustaining support information to support maintenance of life of the infected patient; and
an output step of outputting the information received in the receiving step.

58. A program causing a computer of a mobile terminal to execute:
an activity regulation information receiving step of receiving, from a server configured to generate activity regulation information to regulate an activity of an infected patient of an infectious disease, entry and stay regulation information to regulate entry or stay of the infected patient or entry and exit regulation support information to regulate entry and exit to and from a medical institution for a subject, as the activity regulation information, based on infection determination information indicating presence or degree of infection of the infectious disease in the subject;
a storage step of storing the activity regulation information;
a permission determination step of determining whether or not entry is permitted, whether or not stay is permitted, or whether or not entry and exit are permitted based on the activity regulation information; and
a determination result notification step of sending a notification of a determination result in the permission determination step.
